(19) Europäisches Patentamt
European Patent Office
Office européen des brevets

(11) EP 4 525 816 B1

(12) EUROPEAN PATENT SPECIFICATION

(45) Date of publication and mention of the grant of the patent:
**20.05.2026 Bulletin 2026/21**

(21) Application number: **23725604.5**

(22) Date of filing: **05.05.2023**

(51) International Patent Classification (IPC):
***A61K 8/04*** (2006.01) ***A61K 8/34*** (2006.01)
***A61K 8/37*** (2006.01) ***A61K 8/44*** (2006.01)
***A61K 8/92*** (2006.01) ***A61Q 5/02*** (2006.01)
***A61Q 5/12*** (2006.01) ***A61Q 19/00*** (2006.01)
***A61Q 19/10*** (2006.01)

(52) Cooperative Patent Classification (CPC):
**A61K 8/922; A61K 8/04; A61K 8/342; A61K 8/375; A61K 8/442; A61Q 5/02; A61Q 5/12; A61Q 19/00; A61Q 19/10;** A61K 2800/10

(86) International application number:
**PCT/EP2023/061927**

(87) International publication number:
**WO 2023/222410 (23.11.2023 Gazette 2023/47)**

# (54) WAX DISPERSIONS

WACHSDISPERSIONEN

DISPERSIONS DE CIRE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **17.05.2022 EP 22173762**

(43) Date of publication of application:
**26.03.2025 Bulletin 2025/13**

(73) Proprietor: **BASF SE**
**67056 Ludwigshafen am Rhein (DE)**

(72) Inventors:
- **MAUER, Werner**
  **40589 Duesseldorf (DE)**
- **BECKER, Anke**
  **40589 Duesseldorf (DE)**
- **STEIN, Kirsten**
  **40589 Duesseldorf (DE)**

(74) Representative: **BASF IP Association**
**BASF SE**
**GBI-C006**
**67056 Ludwigshafen (DE)**

(56) References cited:
**WO-A1-2020/229097**
**DE-A1- 102018 215 303**
**US-A1- 2004 037 793**
**US-A1- 2011 247 644**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 525 816 B1

## Description

### Field of the invention

This invention relates to aqueous wax dispersions comprising hydrogenated rape seed oil, method of their preparation and their use as pearlizer and/or conditioner in personal care formulations, especially for the treatment of hair and/or body.

It is known to use wax dispersions containing the wax ethylene glycol distearate (=EGDS) as an excellent pearlizer in personal care products like hair shampoos or body wash formulations to provide them with a pearly, shiny luxurious appearance. However, there is a tendency in the personal care market replacing the wax EDGS in total or in part by natural labelled waxes. Therefore, there is a need to provide wax dispersions, which are do not comprise ethylene oxide and/or ethylene glycol in their chemical structure.

WO03/003991 discloses cosmetic preparations, especially hair preparations, containing semisolid or solid natural oils and glyceryl dioleate for the care of dry or damaged hair, for preventing drying out and split ends and for increasing shine of the hair. The natural oils like hydrogenated vegetable oils or glycerides are used in combination with glyceryl dioleate as an emollient in the hair care preparation. Several natural oils or hydrogenated oils are listed, derived from various plants, e.g. Cremeol® HF-62 derived from a hydrogenated vegetable oil; Cremeol® VP, which is a mixture of vegetable oil, hydrogenated vegetable oil and candelilla wax, or Cremeol® SBE, derived from Shea butter.

WO2020/229097 discloses aqueous cosmetic preparations containing wax particles with an average particle size of from 1.5 to 120 μm, useful as pearlizer and/or opacifier with conditioning properties. The waxes are selected from glycerides or hydrogenated plant oils, waxes derived from plants or animals, synthetic waxes etc. Hydrogenated rapeseed oil is mentioned as one example of the hydrogenated plant oils, but there is no example or any hint how to combine this special wax hydrogenated rapeseed oil with surfactants or emulsifiers to get stable aqueous wax dispersions, which have excellent pearlizing effects and good storage stability.

US2011/0247644 discloses a kit for dyeing and decolorizing keratin containing fibers comprising agents A1, A2 and B, wherein for agent A2 one example A2d comprising Cegesoft HF 62® (= hydrogenated rapeseed oil), Eumulgin VL 75 (mixture of lauryl glucoside+ polyglyceryl-dihydroxystearate+ glycerol), RewotericAM® (Cocoamphoacetate) and Eutanol G ® (= 2-octyldodecanol; so-called guerbet alcohol).

Although there is a great need, in practice it is difficult to replace EDGS in part or in total by natural waxes without having deficiency in performance like appearance, pearlizing effect, viscosity and/or storage stability.

For example, it was observed that aqueous wax dispersions comprising natural waxes like hydrogenated rapeseed oil and amphoteric surfactants show deficiency in their storage stability, especially if the wax dispersion has been stored for weeks and/ or has been exposed to higher or lower temperatures. But wax dispersions itself and personal care products comprising them need to show good storage stability for weeks without segregation or decreasing pearlescent. It was an object of the present invention to provide aqueous wax dispersions based only on natural waxes and showing an excellent pearlizing effect in personal care compositions. This pearlizing effect should at least appear by squeezing or pumping the personal care products out of a bottle, so that the user has a pearlizing shiny personal care product in his hand. The pearlizing effect after pumping or squeezing should be equal or better compared to the effect without.

Additionally, it was an object of the invention to provide aqueous wax dispersions based only on natural waxes and personal care formulations comprising them with a good storage stability at different temperatures and showing a low and stable viscosity range, especially after increasing or lowering the temperatures ("swinging temperature viscosity").

Furthermore, it was an object of the invention to provide aqueous wax dispersions based only on natural waxes having an excellent conditioning effect for their own and in personal care formulations for hair and body for improving conditioning effects. Hair care formulations with such wax dispersions should show improved wet combability, dry combability and/or reduced hair breakage after their use. In body care formulations the use of such wax dispersions should give a soft body feeling.

In one special embodiments, it was an object of the invention to provide wax dispersions with such properties without comprising the typical emulsifier or surfactants compounds containing alkylene oxide like ethylene oxide and/or sulfate groups.

Additionally, it was an object of the invention to provide aqueous wax dispersions based only on natural waxes with extremely small particle sites in the range of 0.1 to 0.5 μ in order to have excellent flow and pumping properties and pearlizing effects.

Additionally, it was an object of the invention to provide aqueous wax dispersions based only on natural waxes which can be used in hair and body care formulations and whereby it is possible to formulate -if desired- hair and body care formulations which do not comprise surfactants comprising ethylene oxide units and/or sulfate groups without losing their advantageous properties.

## Description of the invention

The invention provides aqueous dispersions of natural waxes useful as pearlizer in personal care preparations containing

(a) natural waxes selected from the group comprising 50 to 100 weight (wt)% of hydrogenated rape seed oil,
(b) nonionic emulsifiers selected from the group consisting of linear fatty alcohol and glycerol mono esters
(c) amphoteric and/or zwitterionic surfactants
(d) optional additional nonionic surfactants, different from (b),
(e) optional auxiliaries or additives, including salts, and
(f) water.

In the context of the present invention, "conditioners" are compositions in the field of cosmetic formulations which show a conditioning effect when applied to the hair and/or skin.

In the context of the present invention, compositions have a conditioning effect on the skin when they give rise to a positive skin feel after direct contact with the human skin, which is assessed positively by testers in practice, such as in panel tests with sensory impressions in relation to particular parameters such as "dryness of the skin", "softness of the skin".

In the context of the present invention, the compositions have a conditioning effect on hair when it shows improved combability after treatment thereof. Better combability can occur in the wet and/or dry state, both over the length and in the tips (called disentanglability) of the hair. Conditioning compositions also improve tactile properties of the hair, such as smoothness, softness, suppleness, hair shine, lower electrostatic charge and better shapability or cutting.

In the context of the present invention "hair breakage" mean broken hair fibers.

In the context of the invention "Pearlizer" means compounds or formulations which have a pearlizing effect or pearlizer appearance and are providing cosmetic products with a shimmering, pearluscent and shining effect.

In the context of the present invention, particle size (d50 and/or d90) and particle size distribution in $\mu$m were determined by means of laser diffraction with the Mastersizer® 2000 instrument and the corresponding product description from MALVERN INSTRUMENTS GmbH, Marie-Curie-Straβe 4/1, 71083 Herrenberg, Germany.

In the context of the invention the melting point was measured by using DSC: Differential Scanning Calorimetry.

In the context of the present invention "natural waxes" means substances with a plant or animal origin in nature and a melting point above 25°C, preferred above 50°C and especially above 80°C. The melting point was determined according to ISO 6321 21 °C. The term "natural wax" includes both waxes that are obtained by hydrogenation in parts or totally of animal and / or plant origin in nature and waxes that occur in nature as a wax.

The wax dispersions according to the invention comprise

(a) natural waxes selected from the group comprising 50 to 100 weight (wt)% of hydrogenated rape seed oil.

That mean the natural waxes can be a mixture of natural waxes comprising at least 50 wt% of hydrogenated rape seed oil or hydrogenated rape seed oil is the only the natural wax (100 wt%). Hydrogenated rape seed oil is a triglyceride ester of fatty acids, wherein the unsaturated fatty acids were hydrogenated, i.e transferred to saturated fatty acids.

Typical examples of natural waxes are candelilla wax, carnauba wax, japan wax, esparto grass wax, cork wax, guaruma wax, rice bran wax, sugarcane wax, ouricury wax, shea butter (Butyrospermum Parkii), beeswax, shellac wax, sunflower wax, sumach wax, spermaceti, lanolin (wool wax), uropygial grease, ceresin, ozokerite (earth wax) or hydrogenated jojoba waxes or hydrogenated rape seed oil.

According to the invention at least 50 wt% to 100 wt% of the natural waxes are hydrogenated rape seed oil, especially 100 wt%- based on natural waxes; i.e. it is preferred that the natural waxes (a) are selected from the group consisting of hydrogenated rape seed oil.

Preferred according to the invention is as a natural wax a hydrogenated rape seed oil comprising at least 90 wt% - based on hydrogenated rape seed oil - of triglycerides of a saturated fatty acid mixture consisting of 0-10 wt % C16 fatty acid, 35-45 wt% C18 fatty acid, 5-15 wt % C20 fatty acid and 40-50 wt% C22 fatty acid.

It is possible that up to 10 wt% - based on hydrogenated rape seed oil - by-products are within the hydrogenated rape seed oil like diesters of glycerides or esters of polyglycerides or glycerinesters of unsaturated C16-C22 fatty acids.

However, especially preferred according to the invention is a hydrogenated rape seed oil, comprising more than 90 wt%, more preferred 95-100 wt % - of a triglyceride of fatty acid mixture consisting of (wt%) - based on fatty acids:

| | | |
|---|---|---|
| Palmityl acid: | C16 | approx 5% |
| Stearyl acid | C18 | approx 40% |

(continued)

| | | |
|---|---|---|
| Tetradecyleicosanol acid | C20 | approx 10% |
| Behenyl acid | C22 | approx 45%. |

An example of a commercially available product of hydrogenated rape seed oil is Cegesoft® **HF** 62 from BASF.

The inventive wax dispersions contain the natural waxes (a) preferred in the quantity range from 15 to 40 % by weight, especially preferred from 15-25 %-by weight - based on wax dispersions.

The inventive wax dispersions contain (b) nonionic emulsifiers selected from the group consisting of linear fatty alcohol and glycerol mono esters.

Linear fatty alcohols according to the invention are fatty alcohols, which are not branched in C-C bonding. Examples for linear fatty alcohols are linear fatty alcohols and/or hydroxy fatty alcohols containing 12 to 22 carbon atoms, preferable linear, saturated fatty alcohols and/ or hydroxy fatty alcohols with 16 and/or 18 carbon atoms. Especially preferred is a fatty alcohol mixture of 50 :50 (wt) cetyl alcohol: stearyl alcohol, which is commercially available as Lanette® O from BASF personal Care and Nutrition GmbH

Examples for glycerol mono esters are mono glycerol esters of fatty acids, especially linear and/or branched fatty acids with 12 to 22 carbon atoms and/or linear and/or branched hydroxy fatty acids with 12-18 carbon atoms like hydroxystearic acid monoglyceride, isostearic acid monoglyceride, oleic acid monoglyceride, ricinoleic acid monoglyceride, linoleic acid monoglyceride, linolenic acid monoglyceride, stearic acid monoglyceride and/or oleic stearic acid mono glyceride. Preferred are glycerol mono esters of linear saturated and/or unsaturated fatty acids, especially of glycerol mono stearate and/ or glycerol mono oleate.

Especially preferred within the invention are nonionic emulsifiers b) selected from the group consisting of glycerol mono esters of linear saturated and/or unsaturated fatty acids, especially of glycerol mono stearate and/ or glycerol mono oleate.

Extremely preferred within these products are technical esters of fatty acid monoglycerides which may still contain small amounts of glycerol and/or diglyceride esters and/or triglycerides due to the manufacturing process. Preferred esters are technical mixtures of fatty acid mono glycerides which have a monoglyceride content in the range of 50 to 95 wt%, preferably 60 to 90 wt%. Particularly preferred are mono fatty acid esters of (technical) glycerol and a fatty acid mixture which contains 90 to 100% by weight of oleic acid - based on fatty acid mixture.

Such a technical glycerol mono oleate is for example commercially available as Monomuls® 90/18 from BASF personal Care and Nutrition GmbH.

According to the invention it is preferred that the wax dispersions contain nonionic emulsifiers b) in the quantity range from 0.5 to 5.0 % by weight - based on wax dispersions

According to the invention the wax dispersions contain additionally amphoteric and/or zwitterionic surfactants (c), preferred selected from the group consisting of betaines and/or amphoacetates.

Betaines are known surfactants which are mainly produced by carboxy alkylation, preferably carboxymethylation, of aminic compounds. The starting materials are preferably condensed with halo carboxylic acids or salts thereof, more particularly with sodium chloroacetate. Examples of suitable betaines are the carboxy alkylation products of secondary and, in particular, tertiary amines corresponding to formula (I):

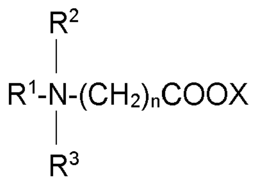

(I)

in which $R^1$ stands for alkyl and/or alkenyl groups containing 6 to 22 carbon atoms, $R^2$ stands for hydrogen or alkyl groups containing 1 to 4 carbon atoms, $R^3$ stands for alkyl groups containing 1 to 4 carbon atoms, n is a number of 1 to 6 and X is an alkali metal and/or alkaline earth metal or ammonium. Typical examples are the carboxymethylation products of hexyl methyl amine, hexyl dimethyl amine, octyl dimethyl amine, decyl dimethyl amine, dodecyl methyl amine, dodecyl dimethyl amine, dodecyl ethyl methyl amine, $C_{12/14}$ cocoalkyl dimethyl amine, myristyl dimethyl amine, cetyl dimethyl amine, stearyl dimethyl amine, stearyl ethyl methyl amine, oleyl dimethyl amine, $C_{16/18}$ tallow alkyl dimethyl amine and technical mixtures thereof.

Other suitable betaines are carboxyalkylation products of amidoamines corresponding to formula (II):

$$R^6CO\text{-}NH\text{-}(CH_2)_m\text{-}\underset{\displaystyle R^5}{\overset{\displaystyle R^4}{N}}\text{-}(CH_2)_nCOOX \qquad (II)$$

in which $R^6CO$ is an aliphatic acyl group containing 6 to 22 carbon atoms and 0 or 1 to 3 double bonds, m is a number of 1 to 3, $R^4$ represents hydrogen or $C_{1-4}$ alkyl groups, $R^5$ represents $C_{1-4}$ alkyl groups, n is a number of 1 to 6 and X is an alkali metal and/or alkaline earth metal or ammonium. Typical examples are reaction products of fatty acids containing 6 to 22 carbon atoms, namely caproic acid, caprylic acid, capric acid, lauric acid, myristic acid, palmitic acid, palmitoleic acid, stearic acid, isostearic acid, oleic acid, elaidic acid, petroselic acid, linoleic acid, linolenic acid, elaeostearic acid, arachic acid, gadoleic acid, behenic acid and erucic acid and technical mixtures thereof, with N,N-dimethyl aminoethyl amine, N,N-dimethyl aminopropyl amine, N,N-diethyl aminoethyl amine and N,N-diethyl aminopropyl amine which are condensed with sodium chloroacetate. It is preferred to use a condensation product of $C_{8/18}$ cocofatty acid-N,N-dimethyl aminopropyl amide with sodium chloroacetate known under the CTFA name of *Cocamidopropyl Betaine.* Betaines distinguished by high purity are particularly preferred; in other words, low-salt betaines with a maximum salt content of 13% by weight, preferably 11% by weight and more particularly 7% by weight - based on active substance - are used. The corresponding salt is dependent on the production of the amphoteric surfactant; in the most common case, it is sodium chloride. In a particularly preferred embodiment, these betaines also have a low content of free fatty acids of at most 4% by weight and preferably at most 3% by weight, based on active substance.

Furthermore, imidazolinium betaines are also included. These substances are also known substances which can be obtained for example by cyclizing condensation of 1 or 2 mol of fatty acid with polyfunctional amines such as, for example, aminoethylethanolamine (AEEA) or diethylenetriamine. The corresponding carboxyalkylation products are mixtures of different open-chain betaines. Typical examples are condensation products of the abovementioned fatty acids with AEEA, preferably imidazolines based on lauric acid or again $C_{12/14}$-coconut fatty acid which are subsequently betainized with sodium chloroacetate.

Suitable cocoamidopropyl betaine are commercially available like Dehyton® PK 45 (supplied by BASF Personal Care and Nutrition GmbH).

It is preferred that the inventive wax dispersion contain said surfactants c) in a quantity range from 10 to 25 wt.% by weight - based on wax dispersion.

According to the invention the wax dispersions may contain optional nonionic surfactants (d), which are different from (b) and are preferred selected from the group consisting of alkyl and/or alkenyl poly glucosides and alkyl and/or alkenyl glucamide.

Alkyl and/or alkenyl polyglycosides are known nonionic surfactants which have in particular the formula (III),

$$RO\text{-}[G]p \qquad (III)$$

in which

- R is an alkyl/alkenyl radical having 6 to 22 carbon atoms,
- G is a sugar radical having five or six carbon atoms and
- p is a number from 1 to 10.

They can be obtained by the relevant methods of preparative organic chemistry. The alkyl/alkenyl polyglycosides can be derived from aldoses or ketoses having 5 or 6 carbon atoms, preferably from glucose. The preferred alkyl polyglycosides are therefore alkyl polyglucosides. The index number p in the general formula (III) specifies the degree of polymerization (DP), i.e. the distribution of mono- and polyglycosides, and is a number between 1 and 10. Whereas p in a given compound must always be an integer and can here in particular assume the values p = 1 to 6, the value p for a particular alkyl/alkenyl polyglycoside is an analytically determined calculated parameter which in most cases is a fraction. Preferably, alkyl/alkenyl polyglycosides are used with an average degree of polymerization p of 1.1 to 3.0. Preference is given to those from a technical applications point of view, for which the degree of polymerization is less than 1.7 and is particularly between 1.2 and 1.7.

The alkyl and/or alkenyl radical R can be derived from primary alcohols having 6 to 22, preferably 6 to 18 carbon atoms. Typical examples are caproic alcohol, caprylic alcohol, decyl alcohol and undecyl alcohol, lauryl alcohol, myristyl alcohol, cetyl alcohol, palmoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol, brassidyl alcohol and also technical grade mixtures thereof.

In the context of the present invention, preference is given in particular to mixtures of alkyl polyglycosides of the formula (III), in which R is derived from primary saturated alcohol mixtures for example from a primary alcohol mixture comprising 10 to 50% by weight of C8/C10 primary alcohol and 50 to 90% by weight of C12 to C16 primary saturated alcohols. Another preference is given for R, which is derived from primary saturated alcohol mixtures comprising 75 to 95% by weight primary higher alcohols with 10 to 22 carbon atoms, especially derivated from fatty acid mixture obtained from coco nut, preferably 12 to 16 carbon atoms.

Suitable products are Plantacare® 2000 and Plantacare® 818, both available by BASF Personal Care Nutrition GmbH.

So-called "alkyl and/or alkenyl glucamide" or "N-alkyl/alkenyl glucamides" are known products and derived from glucuronic acid and fatty acids. Preferred are N-alkylglucamides derived from lauric acid, myristic acid, palmitic acid, palmoleic acid, stearic acid, isostearic acid, oleic acid, elaidic acid, petroselinic acid, linoleic acid, linolenic acid and their technical mixtures. Particular preference is given to the use of a N-methyl glucamides and especially on the basis of a technical C12-C14-coconut fatty acid fraction. Suitable products are Glucopure®Foam or Glucopure® Sense obtainable by Clariant.

According to the invention the wax dispersions contain the nonionic surfactants (d) different from (b) preferred in the quantity range from 0 to 5% by weight - based on wax dispersions. According to one embodiment the inventive wax dispersions do not contain nonionic surfactants d).

According to the invention the wax dispersions may contain other auxiliaries or additives (e), including salts, preferred in the quantity range from 0.1 to 15% by weight - based on wax dispersions.

Examples for other auxiliaries are thickeners, complexing agents, nonaqueous solvents, preservatives, salts and/or pH-adjusters. It is preferred not to add any salt, but dependent on the production of the betaine surfactant (c) low content of salts like sodium chloride or low content free fatty acids may be a possible by-product.

In general, it is advantageous to use preservatives like benzoic acid and/or pH-adjusters like citric acids to adjust the pH as desired. According to the invention it is preferred that only preservatives and/or pH-adjusters and/or salts are present as auxiliaries, but it is possible to add more auxiliaries.

According to the invention the wax dispersions are aqueous dispersions and are characterized in that they contain water (f) in the amount up to 100 % by weight - based on wax dispersions.

According to a preferred embodiment of the invention the wax dispersions contain

a) natural wax selected from the group consisting of hydrogenated rape seed oil comprising at least 90 wt % - based on hydrogenated rape seed oil - triglycerides of fatty acid mixtures comprising 0-10 wt % C16 fatty acid, 35-45 wt% C18 fatty acid, 5-15 wt % C20 fatty acid and 40-50 wt% C22 fatty acid
b) nonionic emulsifiers selected from the group consisting of mono glycerol esters of fatty acids, especially of glycerol mono stearate and/ or glycerol mono oleate
c) amphoteric and/or zwitterionic surfactants selected from the group consisting of betaines and/or amphoacetate and
d) optional nonionic surfactants different from (b) selected from the group consisting of alkyl/alkenyl poly glucosides and alkyl/alkenyl glucamide
(e) other auxiliaries or additives, including salts, and
(f) water.

According to a more preferred embodiment of the invention the aqueous wax dispersions contain

a) 15 - 40 wt % of a natural wax selected from the group consisting of hydrogenated rape seed oil comprising at least 90 wt % - based on hydrogenated rape seed oil - triglycerides of fatty acid mixtures comprising 0-10 wt % C16 fatty acid, 35-45 wt% C18 fatty acid, 5-15 wt % C20 fatty acid and 40-50 wt% C22 fatty acid
b) 0.5 to 5.0 wt % of nonionic emulsifiers selected from the group consisting of mono glycerol esters of fatty acids, especially of glycerol mono stearate and/ or glycerol mono oleate
c) 10.0 to 25.0 wt% of amphoteric and/or zwitterionic surfactants selected from the group consisting of betaines and/or amphoacetate, especially cocoamidopropyl betaine
d) 0.0 to 5.0 wt % nonionic surfactants different from (b) selected from the group consisting of alkyl/alkenyl poly glucosides and alkyl/alkenyl glucamide
e) 0.1 to 15 wt% of other auxiliaries or additives, including salts, especially preservatives and/or pH-adjusters and/or salts and
(f) up to 100 wt % water- based on wax dispersions.

According to the most preferred embodiment of the invention the aqueous wax dispersions are consisting of

a) 15 - 40 wt % of a natural wax selected from the group consisting of hydrogenated rape seed oil comprising at least 90

wt % - based on hydrogenated rape seed oil - triglycerides of fatty acid mixtures comprising 0-10 wt % C16 fatty acid, 35-45 wt% C18 fatty acid, 5-15 wt % C20 fatty acid and 40-50 wt% C22 fatty acid

b) 0.5 to 5.0 wt % of nonionic emulsifiers selected from the group consisting of mono glycerol esters of fatty acids, especially of glycerol mono stearate and/ or glycerol mono oleate

c) 10.0 to 25.0 wt% of amphoteric and/or zwitterionic surfactants selected from the group consisting of betaines and/or amphoacetate, especially cocoamidopropyl betaine

(e) 0.1 to 15 wt% of other auxiliaries or additives, including salts, especially preservatives and/or pH-adjusters and/or salts and

(f) up to 100 wt % water - based on wax dispersions.

Especially preferred are aqueous wax dispersions, which are essentially free of components containing one or more units of ethylene- and/or propylene oxide, i.e. no adducts of one or more ethylene oxide and/or one and/or more propylene oxide were contained as a component to the wax dispersion. This includes that the aqueous wax dispersions are essentially free of components containing ethylene glycol or propylene glycol or derivates like ethylene glycol mono or distearate or ethylene glycol ether compounds.

Additionally, it is preferred, that wax a) as defined, especially said natural wax hydrogenated rape seed oil, is having average particle size d50- measured via laser diffraction by Mastersizer 2000® - from 0.1 to 1.0 $\mu$m, especially 0.1 to 0.2 $\mu$m.

A further object of the invention is a process for the producing aqueous wax dispersions as claimed in claim 1, characterized in that the components (a), (b) and (c) and/or optionally (d) were added to a part of the total water (f), the mixture was heated to about 85 to 95 °C under agitation, cooled down to about 40 to 60 °C before component (e) and the remaining part of water (f) was added.

A further object of the invention is the use of aqueous wax dispersions as claimed in claim 1 or obtained according to the inventive process claim as a pearlizer in personal care compositions for hair and body treatment, especially in amounts of 0.1 to 5 % by weight - calculated as aqueous wax dispersions and based on personal care composition.

A further object of the invention is the use of aqueous wax dispersions as claimed in claim 1 or obtained according to the inventive process claim as conditioner in personal care compositions for hair treatment, especially for improvement of dry and/ or wet combability, preferably in amounts of 0.1-5 % by weight - calculated as aqueous wax dispersions and based on personal care composition.

An additional object of the invention is the use of aqueous wax dispersions as claimed in claim 1 or obtained according to the inventive process claim as conditioner in personal care compositions for body treatment, especially for improving sensoric feeling of the body, preferably in amounts of 0.1-5 % by weight - calculated as aqueous wax dispersions and based on personal care composition.

It is preferred to use the aqueous wax dispersions as claimed in personal care compositions for hair and/or body treatment, which are essentially free from components containing one or more units of ethylene- and/or propylene oxide.

It is preferred to use the aqueous wax dispersions as claimed in personal care compositions for hair and/or body treatment, which are essentially free of surfactants containing sulfate groups.

An additional object of the invention are personal care compositions for hair and/or body treatment comprising

A) 0.1 wt% to 5 wt%, especially 0.25 wt% to 3 wt% of the aqueous dispersions as claimed in claim 1 or obtained according to the process of claim 12 - calculated as aqueous wax dispersions and based on personal care composition - and

B) one or more detersive surfactants selected from the groups consisting of anionic surfactants, nonionic surfactants, amphoteric or zwitterionic surfactants and

C) optional cationic polymers and

D) optional other auxiliaries or additives including salts and water.

Personal care compositions are to be understood here as all compositions known to a person skilled in the art which are exclusively or primarily intended to be applied externally to the human body and/or hair for the cleaning, caring, protection, and maintaining of a good condition, perfuming, changing the appearance or for influencing. Preferably the personal care compositions can be foam baths, shower gels, shower baths, shower milks, shower creams, shampoos, hair masks, hair milks and hair conditioners or hair shampoo soaps or body shampoo soaps.

Component A) are the inventive wax dispersions, which have already been disclosed in detail above.

Component B) are surfactants or surfactant mixtures selected from the group consisting of anionic surfactants, nonionic surfactants, amphoteric or zwitterionic surfactants.

Examples of anionic surfactants are soaps, alkyl benzenesulfonates, alkane sulfonates, olefin sulfonates, alkyl ether sulfonates, glycerol ether sulfonates, ester sulfonates, sulfo fatty acids, alkyl sulfates, fatty alcohol ether sulfates, glycerol ether sulfates, fatty acid ether sulfates, hydroxy mixed ether sulfates, monoglyceride (ether) sulfates, fatty acid

amide (ether) sufates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acid salts such as N-acylamino glutamates or N-acylamino, alkyl polyglucoside sulfates, alkyl- and/or alkenyl polyglucoside carboxylates, alk(en)yl polyglycol ether citrates, protein fatty acid condensates, which are condensation products of $C_8$-$C_{30}$-fatty acids with protein hydrolysates e.g. Lamepon® and alkyl (ether) phosphates. In general, the alkyl- and/or alkenyl residues have 8 to 30, especially 12 to 24, carbon atoms. If the anionic surfactants contain polyglycol ether chains, the polyglycol ether chains may have a conventional homolog distribution, although they preferably have a narrow homolog distribution.

Alkyl ether sulfates ("ether sulfates") are known anionic surfactants which, on an industrial scale, are produced by SO3 or chloro sulfonic acid (CSA) sulfation of fatty alcohol poly glycol ethers or oxoalcohol polyglycol ethers and subsequent neutralization. The ether sulfates may have both a conventional homolog distribution and a narrow homolog distribution. It is particularly preferred to use ether sulfates based on adducts of, on average, 1 to 6 mol and preferably 1 to 3 mol ethylene oxide with technical C12/14 or C12/18 coconut fatty alcohol fractions in the form of their sodium and/or magnesium salts.

Alkyl sulfates ("sulfates") are known anionic surfactants based on alcohols. The products can be obtained commercially. It is preferred to use sulfates based on fatty alcohols having 12 to 18 carbon atoms and very specially sulfates based on technical C12/14 or C12/18 coconut fatty alcohol fractions in the form of their sodium and/or ammonium salts.

Further anionic surfactants useful within the context of the present invention are alpha-sulfo fatty acid di salts according to the formular (IV)

$$R7CH(SO_3M1)COOM2 \qquad (IV),$$

in which the radical R7 is a linear or branched alkyl or alkenyl radical having 6 to 18 carbon atoms and the radicals M1 and M2 - independently of one another - are selected from the group comprising H, Li, Na, K, Ca/2, Mg/2, ammonium and alkanolamines. In this connection, particularly preferred alkanolamines are mono ethanol amine, di ethanol amine, tri ethanolamine and mono isopropanol amine.

In a preferred embodiment, the radical R7 in the formula (IV) is a saturated, linear alkyl radical having 10 to 16 carbon atoms. The radicals M1 and M2 in formula (IV) are preferably selected from the group comprising H (hydrogen) and Na (sodium). The compounds can be prepared by all methods known appropriately to those skilled in the art.

A particularly preferred method of preparation here is the sulfation of the corresponding carboxylic acids. Here, the corresponding carboxylic acid and in particular the corresponding fatty acids are reacted with gaseous sulfur trioxide, the sulfur trioxide being used preferably in an amount such that the molar ratio of $SO_3$ to fatty acid is in the range from 1.0: 1 to 1.1: 1. The crude products obtained in this way, which are acidic sulfation products, are then partially or completely neutralized, preference being given to complete neutralization with aqueous NaOH. If desired, it is also possible to undertake purification steps and/or a bleaching (for adjusting the desired pale color of the products). Preferred alpha-sulfo fatty acid di salts are technical-grade mixtures of the alpha-sulfo fatty acid disalts, which is commercially available as Texapon SFA® from BASF Personal Care Nutrition GmbH.

Further possible anionic surfactants are alk(en)yl polyglycol ether citrates, which are in particular mixtures of mono-, di- and triesters of citric acid and alkoxylated alcohols, particularly preferable to use alkyl poly alkylene glycol ether citrates are based on addition products of from 5 to 10, in particular approximately 7 mol of ethylene oxide on technical grade C12-C18-, in particular C12-C14-fatty alcohol fractions. The polyethylene glycol ethers of lauryl alcohol, laureth-7 citrates, which are obtainable, for example, under the name Plantapon® LC7 (BASF & Personal Care & Nutrition GmbH), are very particularly preferred.

Further anionic surfactants useful within the context of the present invention are N-acyl amino acid salts. N-Acyl amino acid salts are known products and can be made in different ways, for example from the corresponding fatty acyl chlorides and amino acid salts using Schotten-Baumann chemistry or from the corresponding fatty acid and amino alcohol to give a fatty amide, which is then oxidized to give the N-acyl amino acid salt or according US Pat. No. 4,380,646 by contacting an amino carboxylic acid or their metal salts with a low alkyl carboxylic acid esters in the presence of an alkali metal or alkaline earth metal alcoholate or according WO97/03043 by reacting a mono-, di- or triglyceride with an amino acid salt in the presence of a strong base.

Preferred N-acyl amino acid salts are selected from the group sodium cocoyl glycinate, sodium myristyl glycinate, sodium lauryl glycinate, sodium cocoyl glutamate, sodium myristyl glycinate, sodium lauryl glutamate and/or their di salts and/or combinations from them, especially Sodium Cocoyl Glutamate and Disodium Cocoyl Glutamate, which is available as Plantapon® ACG 50 from BASF Personal Care Nutrition GmbH.

Additional useful anionic surfactants within the context of the invention are selected from the group alkyl- and/or alkenyl poly glucoside carboxylates. Alkyl- and/or alkenyl poly glucoside carboxylates are known products and can be made according WO02/090369 from alkyl- and/or alkenyl polyglucoside and Ω-halogen carboxylic acid in presence of

alkali metal hydroxides. Especially preferred is sodium lauryl glucose carboxylate.

Also, suitable as anionic surfactants are protein fatty acid condensates, which can be prepared by all methods known appropriately to those skilled in the art, for example reaction of the corresponding fatty acid chloride with the hydrolyzed protein in alkaline medium. Useful are C12-C24 fatty acid condensation products of protein hydrolysates, especially based on wheat or soja or rice, very specially C12-C24 condensation products of soja protein hydrolysate, which is commercially available as Plantapon® Soy from BASF Personal Care Nutrition GmbH.

Very preferred anionic surfactants according to the invention are selected from the group consisting of alkyl sulfates, N-acyl amino acid salts, alkyl- and/or alkenyl poly glucoside carboxylates and protein fatty acid condensates.

Examples of non-ionic surfactants are

- addition products of from 2 to 50 mol of ethylene oxide and/or 0 to 20 mol of propylene oxide on linear fatty alcohols having 8 to 40 C atoms, on fatty acids having 12 to 40 C atoms and on alkylphenols having 8 to 15 C atoms in the alkyl group;
- $C_{12/18}$-fatty acid mono- and/or diesters of addition products of from 1 to 50 mol of ethylene oxide on glycerol;
- partial esters of glycerol like C12/18-fatty acid mono- and/or diesters of glycerol
- C12/18-fatty acid mono- and/or diesters of mono- and diesters of sorbitol
- alkyl mono- and/or polyglycoside having 8 to 22 carbon atoms in the alkyl radical and optional ethoxylated analogues thereof;
- addition products of from 7 to 60 mol of ethylene oxide on castor oil and/or hydrogenated castor oil;
- polyol and/or polyglycerol esters, such as e.g. polyglycerol diisostearate or polyglycerol dimerate or polyglycerol 12-hydroxystearate;
- addition products of from 2 to 15 mol of ethylene oxide on castor oil and/or hydrogenated castor oil;
- partial esters based on linear, branched, unsaturated or saturated $C_6$-$C_{22}$-fatty acids, ricinoleic acid and 12-hydroxystearic acid with, pentaerythritol, dipentaerythritol, sugar alcohols (e.g. sorbitol), alkyl glucosides (e.g. methyl glucoside, butyl glucoside, lauryl glucoside) and polyglucosides (e.g. cellulose), or mixed esters, such as e.g. glyceryl stearate citrate and glyceryl stearate lactate;
- wool wax alcohols;
- amino-sugars, such as, for example, glucamine.
- polyalkylene glycols.

The addition products of ethylene oxide and/or of propylene oxide on fatty alcohols, fatty acids, alkylphenols, glycerol mono- and diesters and sorbitol mono- and diesters of fatty acids or on castor oil are known, commercially obtainable products. These are homologue mixtures, the average degree of alkoxylation is 1-50 mol of ethylene oxide.

Advantageous compounds from the group of nonionic surfactants are partial esters of polyols, in particular of $C_3$-$C_6$-polyols, such as, for example, glyceryl monoesters, partial esters of pentaerythritol or sugar esters, e.g. sucrose or sorbitol esters.

Preferred partial esters of polyols are mono and/or di esters of fatty acids of glycerol, especially linear and/or branched fatty acids with 12-22 carbon atoms and/or linear and/or branched hydroxy fatty acids with 12-18 carbon atoms like hydroxystearic acid monoglyceride, isostearic acid monoglyceride, oleic acid monoglyceride, ricinoleic acid monoglyceride, linoleic acid monoglyceride, linolenic acid monoglyceride, stearic acid monoglyceride and/or oleic stearic acid mono glyceride. Preferred are glycerol mono esters of linear saturated and/or unsaturated fatty acids, especially of glycerol mono stearate and/ or glycerol mono oleate.

Very preferred is a technical glycerol mono oleate, which was already disclosed as component b) of the inventive wax dispersion in more detail and which is for example commercially available as Monomuls® 90/18 from BASF personal Care and Nutrition GmbH.

Additional useful nonionic surfactants are polyglycerol esters of fatty acids, optional having an additional hydroxyl group like reaction products of poly-12-hydroxystearic acid with polyglycerols which have already been disclosed in more detail as component b) of the inventive wax dispersion and which is marketed, for example, by BASF Personal Care and Nutrition GmbH under the name Dehymuls® PGPH.

Alkyl/ and or alkenyl polyglycosides are particularly useful as nonionic surfactants as component B) in the context of the personal care compositions. Alkyl/and or alkenyl polyglycosides have already been disclosed as an optional component d) of the inventive wax dispersion (see formular (III) and description). Useful nonionic surfactants as component B) are $C_8$-$C_{22}$-alkyl mono- and polyglycosides according to formula (III), preferably with 8 to 18 carbon atoms for the alkyl radical R and a degree of polymerization G within 1.2 to 1.7. Very preferred are the same C8-C22-alkyl mono- and poly glycosides as discussed above as optional component d), i.e. preference is given in particular to mixtures of alkyl polyglycosides of the formula (III), in which R is derived from primary saturated alcohol mixtures for example from a primary alcohol mixture comprising 10 to 50% by weight of C8/C10 primary alcohol and 50 to 90% by weight of C12 to C16 primary saturated alcohols. Another preference is given for R, which is derived from primary saturated alcohol mixtures

comprising 75 to 95% by weight primary higher alcohols with 10 to 22 carbon atoms, especially derivated from fatty acid mixture obtained from coco nut, preferably 12 to 16 carbon atoms. Suitable products are Plantacare® 2000 and Plantacare® 818, both available by BASF Personal Care Nutrition GmbH.

The useful non-ionic surfactants so-called "alkyl glucamide" or "N-alkylglucamides" are known products and have already been discussed in context of optional component d) of the wax dispersion. The same products as mentioned as d) are preferred as non-ionic surfactant B).

Preferred nonionic surfactants B) according to the invention are selected from the group consisting of mono esters of fatty acids with glycerol and alkyl/and or alkenyl polyglycosides and very preferred glycerol mono stearate, glycerol mono oleate and C8-C22 alkyl mono and poly glucosides within a polymerization degree G of 1.2 to 1.7.

Examples of amphoteric and/or zwitterionic surfactants as component B) have already been disclosed in context of component c) as amphoacetate and/or betaines corresponding formular (II) and (I). Preferred betaines corresponding to formular (I) are alkyl dimethyl betaine, especially wherein the alkyl group is based on coconut. Preferred amphoacetates corresponding to formula (II) are condensation products of fatty acid-N,N-dimethyl aminopropyl amide with sodium chloroacetate, especially of coco nut based fatty acids, known under the INCI name of Cocamidopropyl Betaine.

According to one embodiment of the invention the personal care compositions comprising one or more detersive surfactants selected from the group selected of alkyl sulfates, N-acyl amino acid salts, alkyl- and/or alkenyl poly glucoside carboxylates, protein fatty acid condensates, mono esters of fatty acids with glycerol, alkyl/and or alkenyl poly glycosides, alkyl dimethyl betaine, condensation products of fatty acid-N,N-dimethyl aminopropyl amide with sodium chloroacetate, especially selected from the group selected of N-acyl amino acid salts, mono esters of fatty acids with glycerol, alkyl/and or alkenyl poly glycosides, alkyl dimethyl betaine, condensation products of fatty acid-N,N-dimethyl aminopropyl amide with sodium chloroacetate.

It is preferred to use anionic, amphoteric and/or zwitterionic surfactants and/or nonionic surfactants B) in total amounts from 1.0 to 30 wt%, especially 5.0 to 25.0 wt%, - calculated as active matter and based on personal care composition.

Optional component C) are cationic polymers. Useful cationic polymers include cationic guar polymer, a cationic non-guar galactomannan polymer, a cationic tapioca polymer, a cationic copolymer of acrylamide monomers and cationic monomers, and/or a synthetic, non-crosslinked, cationic polymer. Suitable cationic polymers are, for example, cationic cellulose derivatives such as, for example, the quaternized hydroxyethyl cellulose obtainable from Amerchol under the name of Polymer JR 400®, cationic starch, copolymers of diallyl ammonium alts and acrylamides, quaternized vinyl pyrrolidone/vinyl imidazole polymers such as, for example, Luviquat® (BASF), condensation products of polyglycols and amines, quaternized collagen polypeptides such as, for example, Lauryldimonium Hydroxypropyl Hydrolyzed Collagen (Lamequat® L, Grünau), quaternized wheat polypeptides, polyethyleneimine, cationic silicone polymers such as, for example, Amodimethicone, copolymers of adipic acid and dimethyl amino hydroxypropyl diethylenetriamine (Cartaretine®, Sandoz), copolymers of acrylic acid with dimethyl diallyl ammonium chloride (Merquat® 550, Chemviron), polyaminopolyamides and crosslinked water-soluble polymers thereof, cationic chitin derivatives such as, for example, quaternized chitosan, optionally in micro-crystalline distribution, condensation products of dihaloalkyls, for example dibromobutane, with bis-dialkylamines, for example bis-dimethylamino-1,3-propane, cationic guar gum such as, for example, Jaguar® CBS, Jaguar® C-17, Jaguar® C-16 of Celanese, quaternized ammonium salt polymers such as, for example, Mirapol® A-15, Mirapol® AD-1, Mirapol® AZ-1 of Miranol.

Especially, the personal care composition may comprise cationic polymer selected from the group consisting of cationically modified cellulose derivatives, PQ 10, PQ 67, cationically modified guar derivatives, such as, for example, Dehyquart® Guar N, guar hydroxypropyltrimonium chloride, cationic homo- or copolymers based on acrylamide, cationic homo- or copolymers based on vinyl pyrrolidone, cationic homo- or copolymers based on quaternized vinyl imidazole and cationic homo- or copolymers based on methacrylates.

In particular, cationically modified guar derivates are present, preferably guar gum, 2-hydroxy-3-(trimethylammonio)propylether, chloride; INCI: Guar Hydroxypropyl trimonium Chloride; which is commercially available as Dehyquart® Guar N by BASF Personal Care Nutrition GmbH.

In personal care compositions such cationic polymers are preferred used in amounts from 0 to 5 wt%, especially 0.01 to 1.0 wt%, - and in personal care composition for hair treatment like shampoos or conditioners it is preferred to use such cationic polymers in amounts from 0.1 to 0.5 wt%- calculated as active matter and based on the personal care composition.

Optional Components D) are depending on the end-user application of the personal care compositions and may comprise a series of further auxiliaries and additives, such as, for example, water, conditioning agents, pearlizer, viscosity reducers, thickeners, salts, humectant, lipid layer enhancer, superfatting agents, stabilizers, polymers, fats, waxes, lecithins, protein hydrolyates, phospholipids, biogenic active ingredients, UV sunscreen factors, antioxidants, antiperspirants, antidandruff agents, film formers, swelling agents, hydrotropes, solubilizers, preservatives, perfume oils, dyes, and the like.

For reducing the viscosity, the personal care compositions may additionally contain polyols as an optional

component. Suitable polyols preferably contain 2 to 15 carbon atoms and at least two hydroxyl groups and may be modified with nitrogen and/or an amino group. Typical examples are glycerol; alkylene glycols such as, for example, ethylene glycol, diethylene glycol, propylene glycol, butylene glycol, hexylene glycol and polyethylene glycols with an average molecular weight of 100 to 1.000 Dalton; technical polyglycerol mixtures with a degree of self-condensation of 1.5 to 10 such as, for example, technical diglycerol mixtures with a diglycerol content of 40 to 50% by weight; methylol compounds such as, in particular, trimethylol ethane, trimethylol propane, trimethylol butane, pentaerythritol and dipentaerythritol; lower alkyl glucosides, particularly those containing 1 to 8 carbon atoms in the alkyl group, for example methyl and butyl glucoside; sugar alcohols containing 5 to 12 carbon atoms, for example sorbitol or mannitol; amino sugars, for example glucamine; dialcoholamines, such as diethanolamine or 2-aminopropane-1,3-diol.

The polyols are used in quantities of typically 0.1 to 10% by weight, preferably 0.5 to 5% by weight, based on the personal care composition. If larger quantities of polyol, preferably glycerol or ethylene glycol, are used, the personal care compositions are simultaneously stabilized against microbial infestation. Additionally, if glycerol is used, it has an additional effect as a humectant in the personal care compositions.

For personal care compositions using oils like in shower oils or bath oils it is recommended to use for example, Guerbet alcohols based on fatty alcohols containing 6 to 18 , esters of linear C6-22 fatty acids with linear C6-22 fatty alcohols, esters of branched C6-13 carboxylic acids with linear C6-22 fatty alcohols ,esters of linear C6-22 fatty acids with branched alcohols like 2-ethyl hexanol, esters of hydroxycarboxylic acids with linear or branched C6-22 fatty alcohols, triglycerides based on C6-10 fatty acids, liquid mono-/di-/triglyceride mixtures based on C6-18 fatty acids, esters of C6-22 fatty alcohols and/or Guerbet alcohols with aromatic carboxylic acids, vegetable oils, branched primary alcohols, C6-22 fatty alcohol carbonates, Guerbet carbonates, esters of benzoic acid and/or dialkyl ethers containing 6 to 22 carbon atoms per alkyl group.

Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers.

Lipid layer enhancer may be selected from polyethylene glycol glycerol ester, mixtures of dicaprylyl ether and fatty alcohol, polyglycerol poly-12-hydroxystearates and/or mixtures of alkyl poly glucoside and mono esters of glycerol.

The consistency factors mainly used are fatty alcohols or hydroxy fatty alcohols containing 12 to 22 and preferably 16 to 18 carbon atoms and also partial glycerides, fatty acids or hydroxy-fatty acids. A combination of these substances with alkyl poly glucosides and/or fatty acid N-methyl glucamides of the same chain length and/or polyglycerol poly-12-hydroxystearates is preferably used.

Suitable thickeners are, for example, Aerosil types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols® [Goodrich] or Synthalens® [Sigma]), polyacrylamides, polyvinyl alcohol and polyvinyl pyrrolidone, and electrolytes, such as sodium chloride and ammonium chloride. Preferred are thickeners based on xanthan gum like Rheocare® XGN commercially available by BASF Personal Care and Nutrition GmbH.

Metal salts of fatty acids such as, for example, magnesium, aluminium and/or zinc stearate or ricinoleate may be used as stabilizers. Salts like sodium chloride can be incorporated as a by-product or as a rheology modifier.

In the context of the invention, biogenic agents are, for example, tocopherol, tocopherol ace-tate, tocopherol palmitate, ascorbic acid, deoxyribonucleic acid, retinol, bisabolol, allantoin, phytantriol, panthenol, AHA acids, amino acids, ceramides, pseudoceramides, essential oils, plant extracts and vitamin complexes.

Additionally, film formers may be present. Customary film formers are, for example, chitosan, microcrystalline chitosan, quaternized chitosan, polyvinylpyrrolidone, vinylpyrrolidone-vinyl acetate copolymers, polymers of the acrylic acid series, quaternary cellulose derivatives, collagen, hyaluronic acid and salts thereof and similar compounds.

Typical water-soluble additives are, for example, preservatives, water-soluble perfumes, pH regulators, for example buffer mixtures, water-soluble thickeners, for example water-soluble natural or synthetic polymers such as, for example, xanthan gum, hydroxyethyl cellulose, poly-vinyl pyrrolidone or high molecular weight polyethylene oxides.

Suitable antidandruff agents are climbazol, octopirox and zinc pyrithione.

In addition, hydrotropes, for example ethanol, isopropyl alcohol or polyols, may be used to improve flow behavior.

Suitable preservatives are, for example, phenoxyethanol, formaldehyde solution, parabens, pentane diol or sorbic acid, benzoic acid and its salts and the other classes of compounds known by somebody skilled. Preferred is benzoic acid and its salts as preservatives.

Suitable pH-adjuster (= pH regulator) are citric acid and other known acids, which can be buffered. It is preferred to use citric acid to adjust it in the same range as the natural pH of the human skin.

Suitable perfume oils are mixtures of natural and synthetic fragrances. Natural fragrances include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamon, costus, iris, calmus), woods (pinewood, sandalwood, guaiac wood, cedarwood, rosewood), herbs and grasses (tar-ragon,

lemon grass, sage, thyme), needles and branches (spruce, fir, pine, dwarf pine), resins and balsams (galbanum, elemi, benzoin, myrrh, olibanum, opoponax). Typical synthetic perfume compounds are products of the esters, ether, aldehyde, ketone, alcohol and hydrocarbon type. Other suitable perfume oils are essential oils of relatively low volatility which are mostly used as aroma components.

If desired, so-called "Actives", i.e. ingredients which improve properties of hair and/or body, like strengthening hair, reducing hair breakings or improving hair shining or conditioning. Preferred are protein hydrolyzates based on keratin or wheat, rice and/or soya such as the commercially available Nutrilan® Keratin W PP, Gluadin® WLM Benz, Gluadin® WK, Gluadin® WP; Nutrilan® LM, Plantapon® Kera-PLM. It is also possible to add small amounts of free amino acids such as lysine or arginine.

Useful conditioning agents are for example the cationic polymers disclosed as optional component C) alone or in mixture with carrier oils and/or fatty alcohols. Additionally, most of the above mentioned "Actives" have conditioning properties too.

If desired, it is possible to add pearlizer known in theart, e.g. aqueous dispersions based on the wax ethylene glycol distearat.

According to the invention water is one of the other component D).

It is preferred to use these other components D) in amounts up to 100 wt% - based on the personal care composition.

Especially preferred personal care compositions for the treatment of hair and/or body comprising

A) 0.1 wt% to 5 wt%, especially 0.25 wt% to 3 wt%, of the aqueous dispersions as claimed in claim 1 or obtained according to the process of claim 12 - calculated as aqueous wax dispersions and based on personal care composition - and

B) 1.0 to 30 wt%, especially 5.o to 25 wt% - calculated as active matter and based on the personal care composition-, one or more detersive surfactants selected from the groups consisting of anionic surfactants, nonionic surfactants, amphoteric or zwitterionic surfactants and

C) 0 to 5 wt%, especially 0.01 to 1 wt% - calculated as active matter and based on the personal care composition-, cationic polymers and

D) up to 100 wt% other component(s) auxiliaries or additives including salts and water.

Very especially preferred are personal care compositions for the treatment of hair and/or body comprising

0.1 wt% to 5 wt%, especially 0.25 wt% to 3 wt%, of the aqueous dispersions as claimed in claim 1 or obtained according to the process of claim 12 - calculated as aqueous wax dispersions and based on personal care composition - and

1.0 to 30 wt%, especially 5.o to 25 wt% - calculated as active matter and based on the personal care composition-, one or more detersive surfactants selected from the group consisting of alkyl sulfates, N-acyl amino acid salts, alkyl- and/or alkenyl poly glucoside carboxylates, protein fatty acid condensates, mono esters of fatty acids with glycerol, alkyl/and or alkenyl polyglycosides, alkyl dimethyl betaine, condensation products of fatty acid-N,N-dimethyl aminopropyl amide with sodium chloroacetate,

0.01 to 1 wt% - calculated as active matter and based on the personal care composition-, cationic polymers selected from the group of cationically modified guar derivates

up to 100 wt% other auxiliaries or additives including salts and water.

Most preferred are personal care compositions for the treatment of hair and/or body comprising

A) 0.1 wt% to 5 wt%, especially 0.25 wt% to 3 wt%, of the aqueous dispersions as claimed in claim 1 or obtained according to the process of claim 12 - calculated as aqueous wax dispersions and based on personal care composition - and

B) 1.0 to 30 wt%, especially 5.o to 25 wt% - calculated as active matter and based on the personal care composition-, one or more detersive surfactants selected from the group consisting of N-acyl amino acid salts, mono esters of fatty acids with glycerol, alkyl/and or alkenyl polyglycosides, alkyl dimethyl betaine, condensation products of fatty acid-N, N-dimethyl aminopropyl amide with sodium chloroacetate,

C) 0.01 to 1 wt%-calculated as active matter and based on the personal care composition-, cationic polymers selected from the group of cationically modified guar derivates

D) up to 100 wt% other auxiliaries or additives including salts and water.

However, according to the invention it is preferred that none of the compounds of the personal care composition comprising ethylene and/or propylene oxide units.

Moreover, according to the invention it is extremely preferred that none of the surfactants of the personal care

composition comprising sulfate groups.

## Examples

Examples 1: Wax dispersions with hydrogenated rapeseed oil and their properties

The wax dispersions were prepared by mixing all compounds listed in the following table 1a. At first 2/3 of the mentioned water was heated to 90°C, the compounds a) to c) were added in the amount as stated (% weight and calculated as active matter) and stirred. After cooling down to about 60 °C the remaining 1/3 of water was added under agitation. After next cooling down to about 40 °C Na- benzoate and Citric acid (50 wt% solution; amount as is; not calculated as active matter) were added and allowed to cool down to room temperature.

The following compounds were used (%=wt% and calculated as Active Matter = AS):

Cegesoft® HF 62: hydrogenated rapeseed oil; wax; melting point 60-63 °C; hydrogenated triglyceride of fatty acids having the following specification (wt%) for the fatty acids:

| | | |
|---|---|---|
| Palmityl (or Cetyl) acid: | C16:0 | approx 5% |
| Stearyl acid | C18:0 | approx 40% |
| Tetradecyleicosanol acid | C20:0 | approx 10% |
| Behenyl acid | C22:0 | approx 45% |

Monomuls® 90/18: glyceryl mono oleate (nonionic emulsifier)

Lanette® O: Cetearyl Alcohol (50 wt% cetyl- and 50 wt% stearyl alcohol; nonionic emulsifier)

Dehyton® PK 45: aqueous composition with 37 wt% Cocoamidopropylbetain; 7wt % soda chloride and up to 100 wt % water.

**Table 1a Wax Dispersion**

| **Wax Dispersion** | | **1a** | **1b** | **1c** | **1d** | **1e** | **1f** |
|---|---|---|---|---|---|---|---|
| **INCI/Ingredients** | **Product name** | %AS by weight | %AS by weight | %AS by weight | %AS by weight | %AS by weight | %AS by weight |
| Cocoamidopropyl Betaine (c) | Cocoamidopropyl Betaine via Dehyton® PK 45 | 13.0 | 19.5 | 19.5 | 19.5 | 14.8 | - |
| Sodium Cocoamphoacetate(c) | Dehyton® MC | - | - | - | - | - | 13.0 |
| Hydrogenated Rapeseed oil (a) | Cegesoft® HF62 | 18.0 | 20.0 | 20.0 | 20.0 | 20.0 | 18.0 |
| HELIANTHUS ANNUUS SEED CERA, ASCORBYL PALMITATE, TOCOPHEROL | Sunflower Seed wax 6607L | - | - | - | - | - | - |
| Glyceryl mono oleate (b) | Monomuls®90/18 | 2.7 | 3.0 | - | 2.0 | 3.0 | 2.7 |
| Cetearylalcohol(b) | Lanette® O | - | - | 3.0 | - | - | - |
| Lauryl Glucoside | Plantacare® 1200 UP | - | - | - | - | - | - |
| Benzoic acid (d) | | 0.45 | 0.45 | 0.45 | 0.5 | 0.45 | 0.45 |
| Citric acid (50%ig AM) (d) | | 0.7 | 0.7 | 0.7 | 0.7 | 0.7 | 0.7 |
| Water (e) | | Up 100 | Up 100 | Up 100 | Up 100 | Up 100 | Up 100 |
| Viscosity (Brookfield; RVT; spindle 4; 10 rpm; in mPas)<br>20°C /40°C/20°C | | 9080/ 6440/ 7080 | 29520/ 19520/ 21800 | 3080/ 6000/ 9000 | 17600/ 16040/ 16600 | 20040/ 11400/ 12840 | 11000/ 8880/ 10320 |

| **Wax Dispersion** | **1a** | **1b** | **1c** | **1d** | **1e** | **1f** |
|---|---|---|---|---|---|---|
| Viscosity (Brookfield; RVT; spindle 4; 10 rpm; in mPas)<br>20°C /10°C/20°C | 9080/ 9920/ 9000 | 29520/ 32560/ 28800 | 3080/ 3720/ 2640 | 17600/ 16040/ 17800 | 20040/ 21000/ 19720 | 11000/ 8880/ 10320 |
| d 50 µm / d 90 µm | 0,128 / 0,232 | Not determined | Not determined | Not determined | Not determined | Not determined |
| Pearlizing Effect (=PG); White turbidity (=white); see standard formulation Example 2 | PG+; white:+ | PG:++; white:0 | PG+; white:0 | PG+; white:+ | PG:+; white:+ | PG:0; white:0 |

**Table 1a; cont.**

| **Wax Dispersion** | | **Comparison Example V1** | **Comparison Example V2** | **Comparison Example V3** | **Comparison Example V4** |
|---|---|---|---|---|---|
| **INCI/Ingredients** | **Product name** | %AS by weight | %AS by weight | %AS by weight | %AS by weight |
| Cocoamidopropyl Betaine (c) | Cocoamidopropyl Betaine via Dehyton® PK 45 | 19.5 | 13.0 | 13.0 | 13.0 |
| Sodium Cocoamphoacetate(c) | Dehyton® MC | - | - | - | - |
| Hydrogenated Rapeseed oil (a) | Cegesoft® HF62 | 20.0 | - | 18.0 | - |
| HELIANTHUS ANNUUS SEED CERA, ASCORBYL PALMITATE, TOCOPHEROL | Sunflower Seed wax 6607L | - | 18.0 | - | 18.0 |
| Glyceryl mono oleate (b) | Monomuls® 90/18 | - | 2.7 | - | - |
| Cetearylalcohol(b) | Lanette® O | - | - | - | - |
| Lauryl Glucoside | Plantacare® 1200 UP | - | - | 5.2 | 5.2 |
| Benzoic acid (d) | | 0.45 | 0.45 | 0.45 | 0.45 |
| Citric acid (50%ig AM) (d) | | 0.7 | 0.7 | 0.7 | 0.7 |
| Water (e) | | Up 100 | Up 100 | Up 100 | Up 100 |
| Viscosity (Brookfield; RVT; spindle 4; 10 rpm; in mPas) 20°C/40°C/20°C | | Spontaneous separation | Spontaneous separation | Spontaneous separation | Spontaneous separation |
| Viscosity (Brookfield; RVT; spindle 4; 10 rpm; in mPas) 20°C/10°C/20°C | | Spontaneous separation | Spontaneous separation | Spontaneous separation | Spontaneous separation |
| d 50 μm /d 90 μm | | Not determined | Not determined | Not determined | Not determined |
| **Wax Dispersion** | | **Comparison Example V1** | **Comparison Example V2** | **Comparison Example V3** | **Comparison Example V4** |
| Pearlizing Effect (=PG); White turbidity (=white); see standard formulation Example 2 | | Not determined | Not determined | Not determined | Not determined |

Table 1a shows that all inventive wax dispersions have a pearlizing effect, very good storage stabilites without segragation and viscosities which are stable even after swinging temperatures. Inventive wax dispersion 1a has an excellent pearlizing effect and very small wax particle sizes, which is surprising as such pearlizing effects are commonly known for wax dispersions with particle sizes about 10 μm (d 50). The inventive wax dispersion 1 d differs in their amounts of the compounds, but shows the same excellent pearlizing effect as wax dispersion 1a. The inventive wax dispersion 1b with a higher amount of betaine compared to wax dispersion 1a shows an extraordinary pearlizing effect; though wax dispersion 1b) shows a higher viscosity, the viscosity itself is stable, i.e. does not increase after the viscosity swing tests. The inventive wax dispersion 1c) comprises Cetylstearyl alcohol instead of Glyceryl mono oleate and inventive wax dispersion 1f) comprises Sodium Cocoamphoacetate as component c) instead of Cocoamidopropyl Betaine

Comparison example V1 shows that no stable wax dispersion was obtained in the absence of the nonionic emulsifier glyceryl mono oleate (component b)). Comparison example V2 shows that no stable wax dispersion was

obtained on base of the Sunflower Seed wax (component a). In comparison example V3 alkyl polyglucoside was used as a nonionic emulsifier (component b), but the wax dispersion separated spontaneous. The same result could be seen in Comparison example V 4 for a wax dispersion based on Sunflower Seed wax (component a) and polyglucoside as nonionic emulsifier (component b). As all wax dispersions from the comparison examples were not stable no performance tests in standardized shampoo compositions according example 2 have been done.

**Example 2a) to 2f) Standardized shampoo compositions**

The pearlescent effect (PG) and the white turbidity (white) of the wax dispersions listed in table 1a were identified in the following standardized shampoo composition:
weight (wt) % - calculated as active matter;
wt* %: wt % "as the dispersion is" and not calculated as active matter:

| | |
|---|---|
| Deion. Water | 84.7 wt% |
| Texapon® N 70: Sodium Laureth Sulfate | 8.6 wt% |
| Plantacare® 8/18: Coco-Glucosides | 1.6 wt% |
| Dehyton® PK 45: Cocoamidopropyl Betaine | 1.4 wt% |
| Colour Index 16185 Sicomet amaranth (1 % by weight; in water) | 0.1 % |
| Euxyl® K 400®; Fa. Schülke: Preservative | 0.1 wt % |
| NaCl | 1.5 wt% |
| Inventive wax dispersions 1a) to 1f) | each 2 wt* %. |

The shampoo formulation was produced by stirring all components at room temperature (20-23 ° C). After 24 hours an optical assessment of the wax dispersions was made by somebody skilled in the art.
Pearlizing effect (=PG) and white turbidity (=white) have been categorized as follows:

PG: ++ = very good
PG: + = good
PG: - = not good
White: + = good
White: 0 = not good

The results are already listed in table 1a.

**Example 3: Hair Shampoo Formulations with conditioner**

The hair shampoo formulations listed in Table 2a were prepared by the following process: all components were stirred at room temperature (23 °C) until they were completely homogeneous. The wax dispersion according to Example 1a was last added.
weight (wt) % - are based on the hair shampoo and calculated as active matter weight (wt) % - wt* %: wt % "as is" are based on the hair shampoo, but not calculated as active matter; but as the product/dispersion is.
The following further ingredients were used (AS= active matter; or active substance; or SM = solid matter or solid substance) in Example 3 and/or 4:

Dehyquart® Guar N: guar gum, 2-hydroxy-3-(trimethylammonio)propylether, chloride; INCI: Guar Hydroxypropyltrimonium Chloride; AS about 100%
Plantacare® 818: aqueous solution of C8-C18 fatty alcohol polyglycoside; INCI Coco-glucoside; nonionic surfactant; about 52% active matter
Lamesoft® PO 65: mixture of Coco-glucoside with Glycerylmonooleate; INCI:Coco-Glucoside (and) Glyceryloleate; about 65 solid matter
Lamesoft® Balance: wax dispersion of alkyl poly glucoside with hydrogenated castor oil INCI: Coco-Glucoside (and) Hydrogenated Castor Oil; about 43 % solid matter
Plantapon® ACG 50: liquid with Sodium Cocoyl Glutamate and Disodium Cocoyl Glutamate; INCI: Sodium Cocoyl Glutamate; about 40 % soild matter
Plantapon® LGC Sorb: liquid comprising anionic C10-C16 alkyl polyglucoside carboxylate; INCI Sodium Lauryl Glucose Carboxylate (and) Lauryl Glucoside; about 31 % active matter

Plantapon® SF-N: mixture of sodium cocoamphoacetate, glycerine, lauryl glucoside, sodium cocoyl glutamate and sodium glucose carboxylate; INCI Sodium Cocoampho-acetate (and) Glycerin (and) Lauryl Glucoside (and) Sodium Cocoyl Glutamate (and) Sodium Lauryl Glucose Carboxylate; about 40% active matter

Plantasil® Micro: liquid with dicaprylylether, decyl glucoside and glyceryl mono oleate; INCI: Di caprylyl Ether (and) Decyl Glucoside (and) Glyceryl Oleate; about 50 % active matter

Rehocare® XGN: xanthan gum; INCI: Xanthan Gum

Sulfopon® 1216 G: white granules with sodium C12-C18 fatty alcohol sulfate; INCI: Sodium Coco Sulfate

Texapon® ALS Benz: liquid comprising sulfuric acid, mono C10-C16 alkyl esters, ammonium salts INCI: Ammonium Lauryl Sulfate; active matter: about 28 wt%

Dehyton® AB: liquid with C12-C14 alkyldimethyl betaine; INCI: Coco betaine; active matter about 31 wt%

| **Formulation Table 2a** | **INCI** | **1** | **2** | **4** | **5** | **6** | **7** | **Comp C1** | **Comp C2** | Function |
|---|---|---|---|---|---|---|---|---|---|---|
| Dehyquart® Guar N | Guar Hydroxypropyltrimonium Chloride | 0.20 | 0.20 | 0.20 | 0.20 | 0.20 | 0.20 | 0.2 | 0.2 | Conditioning agent |
| Glycerin | Glycerin | x | x | x | x | 3.00 | 3.00 | x | x | Humectant |
| Lamesoft® Balance | Coco-Glucoside (and) Hydrogenated Castor Oil | x | 0.65 | x | x | x | x | x | x | Pearlizer |
| Lamesoft® PO 65 | Coco-glucoside (and)Glyceryl Oleate | x | 1 | 0.65 | x | 0.65 | 0.7 | x | 0.65 | Lipid Layer Enhancer |
| Plantacare® 818 UP | Coco-glucoside | 7.0 | 7.0 | 7.50 | x | 7.50 | 4.3 | 7.0 | 7.5 | Surfactant |
| Plantapon® ACG 50 | Sodium Cocoyl Glutamate | x | x | 6.00 | x | 4.2 | 6.00 | x | 6.0 | Surfactant |
| Plantapon® LGC Sorb | Sodium Lauryl Glucose Carboxylate (and) Lauryl Glucoside | x | x | x | x | 3 | 3 | x | x | Surfactant |
| Plantapon® SF-N | Sodium Co-coamphoacetate (and) Glycerin (and) Lauryl Glucoside (and) Sodium Cocoyl Glutamate (and) Sodium Lauryl Glucose Carboxylate | x | x | x | 6.40 | x | x | x | x | Surfactant |
| Plantasil® Micro | Di caprylyl Ether (and) Decyl Glucoside (and) Glyceryl Oleate | x | x | 1.50 | x | 1.50 | 1.50 | x | 1.5 | Conditioning agent |
| Rheocare® XGN | Xanthan Gum | x | x | 1.20 | x | 1.00 | 1.00 | x | 1.2 | Rheology modifier |
| Sulfopon® 1216 G | Sodium Coco Sulfate | 8.00 | 8.00 | x | x | x | x | 8.0 | x | Surfactant |
| Texapon® ALS Benz | Ammonium Lauryl Sulfate | x | x | x | 4.2 | x | x | x | x | Surfactant |
| Water demin. | Aqua | 78.62 | 76.94 | 75.85 | 77.28 | 61.55 | 55.83 | 81.62 | 78.85 | |
| Perfume; Green Vanille Natura Düllberg | Parfume | 0.30 | 0.30 | 0.30 | 0.30 | 0.30 | 0.30 | 0.3 | 0.3 | Fragrance |
| Sodium Benzoate | Sodium Benzoate | 0.50 | 0.50 | 0.50 | 0.50 | 0.50 | 0.50 | 0.5 | 0.5 | Preservative |
| | | | | | | | | | | |

(continued)

| Formulation Table 2a | INCI | 1 | 2 | 4 | 5 | 6 | 7 | Comp C1 | Comp C2 | Function |
|---|---|---|---|---|---|---|---|---|---|---|
| Citric Acid (50% solution) as is | Citric Acid | 1.55* | 1.55* | 3.30* | 8.12* | 13.6* | 20.67* | 1.55* | 3.3* | pH-adjuster |
| NaCl | Natrium chloride | 0.83 | 0.86 | x | x | x | x | 0.83 | x | salt |
| Wax Dispersion Table 1, example 1a | | 3.00* | 3.00* | 3.00* | 3.00* | 3.00* | 3.00* | x | x | Pearlizer Agent (Invention) |
| Total | | 100 | 100 | 100 | 100 | 100 | 100 | 100 | 100 | |
| **pH adjusted** | pH adjusted | 4.92 | 4.9 | 4.93 | 4.93 | 4.93 | 4.84 | 4.9 | 4.93 | |
| **Viscosity (day 1) [mPas]** | Viscosity (day | 6000 | 6840 | 11000 | 5340 | 8500 | 8800 | n.d | n.d | |
| **Appearance** | | PG | PG | PG | PG | PG | PG | no | no | |
| **Storage: 1week; room temp (RT)** | | 1 | 1 | 1 | 1 | 1 | 1 | n.d | n.d. | |
| **Storage: 1week; (40°C)** | | 1 | 1 | 1 | 1 | 1 | 1 | n.d | n.d. | |
| **Storage:4 weeks; room temp (RT)** | | 1 | 1 | 1 | 1 | 1 | 1 | n.d | n.d | |
| **Wet combing performance (%)** | | 43.4 ± 4.5 | 40.8 ± 4.7 | 45.3 ±8.0 | 45.5± 4.9 | 52.5 ± 6.9 | 47.4 ±5.9 | 63.0± 9.1 | 92.6± 7.1 | |
| **Dry combing performance (%)** | | 20.3 ± 3.6 | 18.8 ± 3.8 | 14.7 ±3.1 | 37.3 ±10.5 | 17.6 ± 3.8 | 18.5 ±3.6 | 28.5± 3.5 | 36.8± 7.9 | |
| **Hair breakage(%)** | | 1.5 | n.d. | 0.7 | n.d. | n.d. | n.d. | 14.9 | 20.0 | |
| **n.d = not determined** | | | | | | | | | | |

All hair shampoo formulations in table 2a comprising the wax claimed dispersions show a pearlizing effect, which is stable for a long period of time (1 to 4 weeks) at room temperature and at higher temperatures without any separation (=identified as "1").

Additionally, the hair shampoo formulations have excellent conditioning properties and reduces hair break significantly.

The conditioning properties can be shown in low figures in (%) measured as residual wet combability (=wet combining properties) and/or residual dry combability (= dry combining properties), which was determined as follows:

Preparation of Hair Strands for the Test

Ten hair strands (medium-dark brown Caucasian hair (12 cm / 1 g, International Hair Importers & Products, USA) per sample were cleansed by incubation in 6 % sodium lauryl ether sulfate (ac), pH 6.5, for 30 minutes, followed by rinsing and submerging three times in water for two minutes each. Then the hair was bleached with 5 % hydrogen peroxide (pH 9.4) for 20 minutes, followed by extensive rinsing. Finally, the hair strands were dried for 30 minutes hanging in a flow of warm air (approximately 55 °C). The preparation described was done by an automated system.

Treatment of the hair tresses with formulations 1) to 7) listed in table 2a

In an automated method, a measurement before and after the treatment of the hair with the inventive hair shampoo formulations 1) to 7) and for comparison with C1) and C2) was done. For this purpose, the hair was treated with 0.125 g/g of hair for 5 min.

In the automated system of the wet combing apparatus, the treated hair tresses were rinsed under standard conditions (38°C, 1 l/minute) for 1 minute and measured.

For the determination of dry combability, the treated hair tresses were stored at 30°C and 40% relative air humidity for 16 hours.

Repeated Combing of Hair Strands

The hair strands were combed 50,000 times (45 rpm) at 30 °C and 40 % relative humidity using a special combing device

For each determination, 10 hair tresses were tested and the combing forces were measured over 23 comb movements (3rd to 23rd).

% residual combability was calculated as follows:

Residual combability (%) = (residual combing work after product treatment: combing work before product treatent) × 100.

Evaluation of Hair Breakage

After 50,000 combing strokes broken hair fibers were collected in drawers. Then fibers longer than the diameter of the petri dish (about 9 cm) were sorted out, and the remaining fibers were weighed. Hair breakage is quantified as ratio of weight of broken fibers to weight of the whole hair strand.

Test of Significance

Statistical significance ($p< =0.05$) of differences between the formulations and in comparison to untreated hair was calculated using Tukey's test. Tukey's test is a single-step multiple comparison procedure and statistical test used in conjunction with an ANOVA (analysis of variance) to find which means are significantly different from one another. If the ANOVA test indicates significant differences, a Tukey's test is performed. It compares all possible pairs of means and is based on a studentized range distribution (this distribution is similar to the distribution of t from the t.test). The test compares the means of every treatment to the means of every other treatment. It is applied simultaneously to the set of all pair wise comparisons and identifies where the difference between two means is greater than the standard error would be expected to allow. The threshold of the test is the HSD-range (Honestly Significant Difference).

The HSD-range is determined by the signal-to-noise ratio, and the number of measurements. The difference between two samples has to be greater than the calculated HSD value to be significantly different at a 95 % probability.

It becomes clear from table 2a) that all hair shampoo formulations comprising the wax dispersion according to table 1a example 1a) show better residual wet and dry combability than formulations without. This is also true in the presence of cationic polymers, which are the "typical" compound for getting conditioning properties. For example, shampoo 1 with the wax dispersion is significant better as the comparison shampoo C1 or shampoo 4 is extremely better than comparison shampoo C2 regarding the dry combability and the wet combability and the hair breakage. As a

result, table 2a shows that the wax dispersion has additionally conditioning properties itself and/or boost the conditioning properties of cationic modified polymers in hair shampoo formulation.

**Example 4: Body Wash Formulations without conditioner**

The body wash formulations were prepared as the hair care formulations in example 3. Wax dispersion according to Example 1a was last added. The composition and their properties are shown in table 2b, whereby weight (wt) % - are based on the hair shampoo and calculated as active matter weight (wt) % -
wt* %: wt % "as is" are based on the hair shampoo, but not calculated as active matter; but as the product/dispersion is

| **Formulation Table 2b** | **INCI** | **8** | **9** | **Function** |
|---|---|---|---|---|
| Dehyton® AB 30 | Coco betaine | x | 4,00 | Surfactant |
| Dehyton® PK 45 | Cocamidopropyl Betaine | 6,30 | x | Surfactant |
| Glycerin | Glycerin | 2,00 | x | Humectant |
| Lamesoft® PO 65 | Coco-glucoside (and) Glyceryl Oleate | 0,65 | 0,65 | Lipid Layer Enhancer |
| Plantacare® 818 UP | Coco-Glucoside | 1,05 | 5,00 | Surfactant |
| Plantapon® SF-N | Sodium Cocoamphoacetate (and) Glycerin (and) Lauryl Glucoside (and) Sodium Cocoyl Glutamate (and) Sodium Lauryl Glucose Carboxylate | 4,4 | x | Surfactant |
| Rheocare® XGN | Xanthan Gum | 0,6 | 0,6 | Rheologie Modifier |
| Water demin. | Aqua (water) | 79.9 | 86.23 | |
| Perfume;Cottan Touch | Parfum | 0,3 | 0,3 | Fragrance |
| Sodium Benzoate | Sodium Benzoate | 0,5 | 0,5 | Preservative |
| Citric Acid **(50% solution)(*)** | Citric Acid* | 1,30* | 0,72* | pH-adjuster |
| Wax Dispersion Table 1, example 1a | | 3,00* | 2,00* | Pearlizer (Invention) |
| Total | | 100 | 100 | |
| **pH adjusted** | pH adjusted | 4,93 | 4,89 | |
| **Viscosity (day 1) [mPas]** | Viscosity (day | 5200 | 5500 | |
| **Appearance (Pearlizing effect=PG)** | | PG | PG | |
| **Storage (1 week)/-room temp. RT** | | 1 | 1 | |
| **Storage (1 week)/40°C** | | 1 | 1 | |
| **Storage (4 week)/RT** | | 1 | 1 | |
| **Storage (4 weeks)/40°C** | | 1 | 1 | |

All body wash formulations show a pearlizing effect, which is stable for a long period of time (1 to 4 weeks) at room temperature and at higher temperatures (1 to 4 weeks at 40 °C) without any separation (=identified as "1").

Additionally, the body wash formulations have excellent sensoric properties and give a skin feeling of a rich care.

## Claims

1. Aqueous dispersions of natural waxes useful as pearlizer in personal care preparations containing

   (a) natural waxes selected from the group comprising 50 to 100 weight (wt)% of hydrogenated rape seed oil,
   (b) nonionic emulsifiers selected from the group consisting of linear fatty alcohol and glycerol mono esters
   (c) amphoteric and/or zwitterionic surfactants
   (d) optional additional nonionic surfactants, different from (b),
   (e) optional auxiliaries or additives, including salts, and
   (f) water.

2. Aqueous dispersions as claimed in claim 1, **characterized in that** they contain (a) natural waxes selected from the group consisting of hydrogenated rape seed oil, or
   **characterized in that** they contain (a) natural waxes selected from the group consisting of hydrogenated rape seed oil comprising at least 90 wt% - based on hydrogenated rape seed oil - of triglycerides of a fatty acid mixture consisting of 0-10 wt % C16 fatty acid, 35-45 wt% C18 fatty acid, 5-15 wt % C20 fatty acid and 40-50 wt% C22 fatty acid.

3. Aqueous dispersions as claimed in claim 1, **characterized in that** they contain natural waxes (a) in the quantity range from 15 to 40 % by weight - based on wax dispersions.

4. Aqueous dispersions as claimed in claim 1, **characterized in that** they contain nonionic emulsifiers b) selected from the group consisting of mono glycerol esters of linear saturated and/or unsaturated fatty acids, especially of glycerol mono stearate and/ or glycerol mono oleate, and/or
   **characterized in that** they contain nonionic emulsifiers b) in the quantity range from 0.5 to 5.0 % by weight - based on wax dispersions.

5. Aqueous dispersions as claimed in claim 1, **characterized in that** they contain amphoteric and/or zwitterionic surfactants (c) selected from the group consisting of betaines and/or amphoacetates, and/or
   **characterized in that** they contain amphoteric and/or zwitterionic surfactants (c) in the quantity range from 10 to 25% by weight, - based on wax dispersions.

6. Aqueous dispersions as claimed in claim 1, **characterized in that** they contain additional nonionic surfactants (d) different from (b) selected from the group consisting of alkyl and/or alkenyl poly glucosides and alkyl and/or alkenyl glucamide, and/or
   **characterized in that** they contain additional nonionic surfactants (d) different from (b) in the quantity range from 0 to 5% by weight - based on wax dispersions.

7. Aqueous dispersions as claimed in claim 1, **characterized in that** they contain other auxiliaries or additives (e), including salts, in the quantity range from 0.1 to 15% by weight - based on wax dispersions, and/or
   **characterized in that** they contain water (f) in the amount up to 100 % by weight - based on wax dispersions.

8. Aqueous dispersions as claimed in claim 1, **characterized in that** they contain

   a) natural wax selected from the group consisting of hydrogenated rape seed oil comprising at least 90 wt %- based on hydrogenated rape seed oil- triglycerides of fatty acid mixtures comprising 0-10 wt % C16 fatty acid, 35-45 wt% C18 fatty acid, 5-15 wt % C20 fatty acid and 40-50 wt% C22 fatty acid
   b) nonionic emulsifiers selected from the group consisting of mono glycerol esters of fatty acids, especially of glycerol mono stearate and/ or glycerol mono oleate
   c) amphoteric and/or zwitterionic surfactants selected from the group consisting of betaines and/or amphoacetate and
   d) optional nonionic surfactants different from (b) selected from the group consisting of alkyl/alkenyl poly glucosides and alkyl/alkenyl glucamide
   (e) other auxiliaries or additives, including salts, and
   (f) water

   or **characterized in that** they contain

   a) 15 - 40 wt % of a natural wax selected from the group consisting of hydrogenated rape seed oil comprising at

least 90 wt % - based on hydrogenated rape seed oil- triglycerides of fatty acid mixtures comprising 0-10 wt % C16 fatty acid, 35-45 wt% C18 fatty acid, 5-15 wt % C20 fatty acid and 40-50 wt% C22 fatty acid
b) 0.5 to 5.0 wt % of nonionic emulsifiers selected from the group consisting of mono glycerol esters of fatty acids, especially of glycerol mono stearate and/ or glycerol mono oleate
c) 10.0 to 25.0 wt% of amphoteric and/or zwitterionic surfactants selected from the group consisting of betaines and/or amphoacetate, especially cocoamidopropyl betaine
d) 0.0 to 5.0 wt % nonionic surfactants different from (b) selected from the group consisting of alkyl/alkenyl poly glucosides and alkyl/alkenylglucamide
e) 0.1 to 15 wt% of other auxiliaries or additives, including salts, especially preservatives and/or pH-adjusters and/or salts and
(f) up to 100 wt % water - based on wax dispersions.

**9.** Aqueous wax dispersions as claimed in claim 1, **characterized in that** they are essentially free of components containing one or more units of ethylene- and/or propylene oxide.

**10.** Aqueous dispersions as claimed in claim 1, **characterized in that** they are consisting of

a) 15 - 40 wt % of a natural wax selected from the group consisting of hydrogenated rape seed oil comprising at least 90 wt % - based on hydrogenated rape seed oil - triglycerides of fatty acid mixtures comprising 0-10 wt % C16 fatty acid, 35-45 wt% C18 fatty acid, 5-15 wt % C20 fatty acid and 40-50 wt% C22 fatty acid
b) 0.5 to 5.0 wt % of nonionic emulsifiers selected from the group consisting of mono glycerol esters of fatty acids, especially of glycerol mono stearate and/ or glycerol mono oleate
c) 10.0 to 25.0 wt% of amphoteric and/or zwitterionic surfactants selected from the group consisting of betaines and/or amphoacetate, especially cocoamidopropyl betaine
(e) 0.1 to 15 wt% of other auxiliaries or additives, including salts, especially preservatives and/or pH-adjusters and/or salts and
(f) up to 100 wt % water- based on wax dispersions.

**11.** Aqueous wax dispersions as claimed in claim 1, **characterized in that** the natural wax hydrogenated rape seed oil having an average particle size d50- measured via laser diffraction by Mastersizer 2000® - from 0.1 to 1.0 μm, especially 0.1 to 0.2 μm.

**12.** A process for the producing aqueous wax dispersions as claimed in claim 1, **characterized in that** after the components (a), (b) and (c) and/or optionally (d) were added to a part of the total water (f) the mixture was heated to about 85 to 95 °C under agitation, cooled down to about 40 to 60 °C before component (e) and the remaining part of water (f) was added.

**13.** Use of aqueous wax dispersions as claimed in claim 1 or 12 as a pearlizer in personal care compositions for hair and/or body treatment, especially in amounts of 0.1-5 % by weight-calculated as aqueous wax dispersions and based on personal care composition, or

use of aqueous wax dispersions as claimed in claim 1 or 12 as a conditioner in personal care compositions for hair treatment, especially for improvement of dry and/ or wet combability, and/or hair breakage, preferably in amounts of 0.1-5 % by weight - calculated as aqueous wax dispersions and based on personal care composition, or
use of aqueous wax dispersions as claimed in claim 1 or 12 as a conditioner in personal care compositions for body treatment, especially for improving sensoric feeling of the body, preferably in amounts of 0.1-5 % by weight - calculated as aqueous wax dispersions and based on personal care composition.

**14.** Use of aqueous wax dispersions as claimed in claim 13 in personal care compositions for hair and/or body treatment, which are essentially free from components containing one or more units of ethylene- and/or propylene oxide, or use of aqueous wax dispersions as claimed in claim 13 in personal care compositions for hair and/or body treatment, which are essentially free of surfactants containing sulfate groups.

**15.** Personal care compositions for hair and/or body treatment comprising

A) 0.1 wt% to 5 wt%, especially 0.25 wt% to 3 wt% of the aqueous wax dispersions as claimed in claim 1 or obtained according to the process of claim 12 - calculated as aqueous wax dispersions and based on personal care composition - and

B) one or more detersive surfactants selected from the groups consisting of anionic surfactants, nonionic surfactants, amphoteric or zwitterionic surfactants and
C) optional cationic polymers and
D) optional other auxiliaries or additives including salts and water.

**Patentansprüche**

1. Wässrige Dispersionen natürlicher Wachse, geeignet als Perlglanzmittel in Körperpflegezubereitungen, enthaltend

   (a) natürliche Wachse, ausgewählt aus der Gruppe umfassend 50 bis 100 Gew.-% hydriertes Rapsöl,
   (b) nichtionische Emulgatoren, ausgewählt aus der Gruppe bestehend aus linearen Fettalkoholen und Glycerinmonoestern,
   (c) amphotere und/oder zwitterionische Tenside,
   (d) optionale zusätzliche nichtionische Tenside, verschieden von (b),
   (e) optionale Hilfs- oder Zusatzstoffe, einschließlich Salzen, und
   (f) Wasser.

2. Wässrige Dispersionen nach Anspruch 1, **dadurch gekennzeichnet, dass** sie

   (a) natürliche Wachse enthalten, ausgewählt aus der Gruppe bestehend aus hydriertem Rapsöl, oder

   **dadurch gekennzeichnet, dass** sie

   (a) natürliche Wachse enthalten, ausgewählt aus der Gruppe bestehend aus hydriertem Rapsöl, wobei das hydrierte Rapsöl mindestens 90 Gew.-%, bezogen auf hydriertes Rapsöl, an Triglyceriden eines Fettsäuregemisches bestehend aus 0-10 Gew.-% C16-Fettsäuren, 35-45 Gew.-% C18-Fettsäuren, 5-15 Gew.-% C20-Fettsäuren und 40-50 Gew.-% C22-Fettsäuren aufweist.

3. Wässrige Dispersionen nach Anspruch 1, **dadurch gekennzeichnet, dass** sie natürliche Wachse (a) in einem Mengenbereich von 15 bis 40 Gew.-%, bezogen auf die Wachsdispersionen, enthalten.

4. Wässrige Dispersionen nach Anspruch 1, **dadurch gekennzeichnet, dass** sie nichtionische Emulgatoren (b) enthalten, ausgewählt aus der Gruppe bestehend aus Glycerinmonoestern linearer gesättigter und/oder ungesättigter Fettsäuren, insbesondere Glycerinmonostearat und/oder Glycerinmonooleat, und/oder
   **dadurch gekennzeichnet, dass** sie nichtionische Emulgatoren (b) in einem Mengenbereich von 0,5 bis 5,0 Gew.-%, bezogen auf die Wachsdispersionen, enthalten.

5. Wässrige Dispersionen nach Anspruch 1, **dadurch gekennzeichnet, dass** sie amphotere und/oder zwitterionische Tenside (c) enthalten, ausgewählt aus der Gruppe bestehend aus Betainen und/oder Amphoacetaten, und/oder
   **dadurch gekennzeichnet, dass** sie amphotere und/oder zwitterionische Tenside (c) in einem Mengenbereich von 10 bis 25 Gew.-%, bezogen auf die Wachsdispersionen, enthalten.

6. Wässrige Dispersionen nach Anspruch 1, **dadurch gekennzeichnet, dass** sie zusätzliche nichtionische Tenside (d), verschieden von (b), enthalten, ausgewählt aus der Gruppe bestehend aus Alkyl- und/oder Alkenylpolyglycosiden und Alkyl- und/oder Alkenylglucamiden, und/oder
   **dadurch gekennzeichnet, dass** sie zusätzliche nichtionische Tenside (d), verschieden von (b), in einem Mengenbereich von 0 bis 5 Gew.-%, bezogen auf die Wachsdispersionen, enthalten.

7. Wässrige Dispersionen nach Anspruch 1, **dadurch gekennzeichnet, dass** sie weitere Hilfs- oder Zusatzstoffe (e), einschließlich Salzen, in einem Mengenbereich von 0,1 bis 15 Gew.-%, bezogen auf die Wachsdispersionen, enthalten, und/oder
   **dadurch gekennzeichnet, dass** sie Wasser (f) in einer Menge von bis zu 100 Gew.-%, bezogen auf die Wachsdispersionen, enthalten.

8. Wässrige Dispersionen nach Anspruch 1, **dadurch gekennzeichnet, dass** sie enthalten

   (a) ein natürliches Wachs, ausgewählt aus der Gruppe bestehend aus hydriertem Rapsöl mit mindestens 90

Gew.-%, bezogen auf hydriertes Rapsöl, an Triglyceriden eines Fettsäuregemisches bestehend aus 0-10 Gew.-% C16-Fettsäuren, 35-45 Gew.-% C18-Fettsäuren, 5-15 Gew.-% C20-Fettsäuren und 40-50 Gew.-% C22-Fettsäuren,
(b) nichtionische Emulgatoren, ausgewählt aus der Gruppe bestehend aus Glycerinmonoestern von Fettsäuren, insbesondere Glycerinmonostearat und/oder Glycerinmonooleat,
(c) amphotere und/oder zwitterionische Tenside, ausgewählt aus der Gruppe bestehend aus Betainen und/oder Amphoacetaten, und
(d) optionale nichtionische Tenside, verschieden von (b), ausgewählt aus der Gruppe bestehend aus Alkyl-/Alkenylpolyglycosiden und Alkyl-/Alkenylglucamiden,
(e) weitere Hilfs- oder Zusatzstoffe, einschließlich Salzen, und
(f) Wasser,

oder **dadurch gekennzeichnet, dass** sie enthalten

(a) 15-40 Gew.-% eines natürlichen Wachses, ausgewählt aus der Gruppe bestehend aus hydriertem Rapsöl mit mindestens 90 Gew.-%, bezogen auf hydriertes Rapsöl, an Triglyceriden eines Fettsäuregemisches bestehend aus 0-10 Gew.-% C16-Fettsäuren, 35-45 Gew.-% C18-Fettsäuren, 5-15 Gew.-% C20-Fettsäuren und 40-50 Gew.-% C22-Fettsäuren,
(b) 0,5-5,0 Gew.-% nichtionischer Emulgatoren, ausgewählt aus der Gruppe bestehend aus Glycerinmonoestern von Fettsäuren, insbesondere Glycerinmonostearat und/oder Glycerinmonooleat,
(c) 10,0-25,0 Gew.-% amphoterer und/oder zwitterionischer Tenside, ausgewählt aus der Gruppe bestehend aus Betainen und/oder Amphoacetaten, insbesondere Cocamidopropylbetain,
(d) 0,0-5,0 Gew.-% nichtionischer Tenside, verschieden von (b), ausgewählt aus der Gruppe bestehend aus Alkyl-/Alkenylpolyglycosiden und Alkyl-/Alkenylglucamiden,
(e) 0,1-15 Gew.-% weiterer Hilfs- oder Zusatzstoffe, einschließlich Salzen, insbesondere Konservierungsmittel und/oder pH-Einstellmittel und/oder Salze, und
(f) bis zu 100 Gew.-% Wasser, bezogen auf die Wachsdispersionen.

**9.** Wässrige Wachsdispersionen nach Anspruch 1, **dadurch gekennzeichnet, dass** sie im Wesentlichen frei von Komponenten sind, die eine oder mehrere Einheiten von Ethylenoxid und/oder Propylenoxid enthalten.

**10.** Wässrige Dispersionen nach Anspruch 1, **dadurch gekennzeichnet, dass** sie bestehen aus

(a) 15-40 Gew.-% eines natürlichen Wachses, ausgewählt aus der Gruppe bestehend aus hydriertem Rapsöl mit mindestens 90 Gew.-%, bezogen auf hydriertes Rapsöl, an Triglyceriden eines Fettsäuregemisches bestehend aus 0-10 Gew.-% C16-Fettsäuren, 35-45 Gew.-% C18-Fettsäuren, 5-15 Gew.-% C20-Fettsäuren und 40-50 Gew.-% C22-Fettsäuren,
(b) 0,5-5,0 Gew.-% nichtionischer Emulgatoren, ausgewählt aus der Gruppe bestehend aus Glycerinmonoestern von Fettsäuren, insbesondere Glycerinmonostearat und/oder Glycerinmonooleat,
(c) 10,0-25,0 Gew.-% amphoterer und/oder zwitterionischer Tenside, ausgewählt aus der Gruppe bestehend aus Betainen und/oder Amphoacetaten, insbesondere Cocamidopropylbetain,
(e) 0,1-15 Gew.-% weiterer Hilfs- oder Zusatzstoffe, einschließlich Salzen, insbesondere Konservierungsmittel und/oder pH-Einstellmittel und/oder Salze, und
(f) bis zu 100 Gew.-% Wasser, bezogen auf die Wachsdispersionen.

**11.** Wässrige Wachsdispersionen nach Anspruch 1, **dadurch gekennzeichnet, dass** das natürliche Wachs hydriertes Rapsöl eine mittlere Partikelgröße d50, gemessen mittels Laserbeugung mit einem Mastersizer 2000®, von 0,1 bis 1,0 μm, insbesondere von 0,1 bis 0,2 μm, aufweist.

**12.** Verfahren zur Herstellung wässriger Wachsdispersionen nach Anspruch 1, **dadurch gekennzeichnet, dass** nach dem Zugeben der Komponenten (a), (b) und (c) und/oder optional (d) zu einem Teil des gesamten Wassers (f) das Gemisch unter Rühren auf etwa 85 bis 95 °C erhitzt wird, anschließend auf etwa 40 bis 60 °C abgekühlt wird, bevor die Komponente (e) und der verbleibende Teil des Wassers (f) zugegeben werden.

**13.** Verwendung wässriger Wachsdispersionen nach Anspruch 1 oder 12 als Perlglanzmittel in Körperpflegezusammensetzungen zur Haar- und/oder Körperbehandlung, insbesondere in Mengen von 0,1-5 Gew.-%, berechnet als wässrige Wachsdispersionen und bezogen auf die Körperpflegezusammensetzung, oder

Verwendung wässriger Wachsdispersionen nach Anspruch 1 oder 12 als Conditioner in Körperpflegezusammensetzungen zur Haarbehandlung, insbesondere zur Verbesserung der trockenen und/oder nassen Kämmbarkeit und/oder zur Reduktion von Haarbruch, vorzugsweise in Mengen von 0,1-5 Gew.-%, berechnet als wässrige Wachsdispersionen und bezogen auf die Körperpflegezusammensetzung, oder
Verwendung wässriger Wachsdispersionen nach Anspruch 1 oder 12 als Conditioner in Körperpflegezusammensetzungen zur Körperbehandlung, insbesondere zur Verbesserung des sensorischen Hautgefühls, vorzugsweise in Mengen von 0,1-5 Gew.-%, berechnet als wässrige Wachsdispersionen und bezogen auf die Körperpflegezusammensetzung.

**14.** Verwendung wässriger Wachsdispersionen nach Anspruch 13 in Körperpflegezusammensetzungen zur Haar- und/oder Körperbehandlung, die im Wesentlichen frei von Komponenten sind, die eine oder mehrere Einheiten von Ethylenoxid und/oder Propylenoxid enthalten, oder
Verwendung wässriger Wachsdispersionen nach Anspruch 13 in Körperpflegezusammensetzungen zur Haar- und/oder Körperbehandlung, die im Wesentlichen frei von Tensiden sind, die Sulfatgruppen enthalten.

**15.** Körperpflegezusammensetzungen zur Haar- und/oder Körperbehandlung, enthaltend

(A) 0,1-5 Gew.-%, insbesondere 0,25-3 Gew.-%, der wässrigen Wachsdispersionen nach Anspruch 1 oder hergestellt gemäß dem Verfahren nach Anspruch 12, berechnet als wässrige Wachsdispersionen und bezogen auf die Körperpflegezusammensetzung, und
(B) ein oder mehrere reinigende Tenside, ausgewählt aus der Gruppe bestehend aus anionischen Tensiden, nichtionischen Tensiden, amphoteren oder zwitterionischen Tensiden, sowie
(C) optionale kationische Polymere und
(D) optionale weitere Hilfs- oder Zusatzstoffe, einschließlich Salzen und Wasser.

## Revendications

**1.** Dispersions aqueuses de cires naturelles utilisables comme agent perlant dans des préparations de soins personnels, comprenant :

(a) des cires naturelles sélectionnées dans le groupe comprenant 50 à 100 % en poids (pds) d'huile de colza hydrogénée,
(b) des émulsifiants non ioniques sélectionnés dans le groupe constitué d'alcools gras linéaires et de monoesters de glycérol,
(c) des tensioactifs amphotères et/ou zwitterioniques,
(d) des tensioactifs non ioniques additionnels optionnels, différents de (b),
(e) des auxiliaires ou additifs optionnels, y compris des sels, et
(f) de l'eau.

**2.** Dispersions aqueuses selon la revendication 1, **caractérisées en ce qu'**elles contiennent

(a) des cires naturelles sélectionnées dans le groupe constitué d'huile de colza hydrogénée,

ou **caractérisées en ce qu'**elles contiennent

(a) des cires naturelles sélectionnées dans le groupe constitué d'huile de colza hydrogénée comprenant au moins 90 % en poids, sur la base de l'huile de colza hydrogénée, de triglycérides d'un mélange d'acides gras constitué de 0 à 10 % en poids d'acide gras C16, 35 à 45 % en poids d'acide gras C18, 5 à 15 % en poids d'acide gras C20 et 40 à 50 % en poids d'acide gras C22.

**3.** Dispersions aqueuses selon la revendication 1, **caractérisées en ce qu'**elles contiennent les cires naturelles (a) dans une plage quantitative de 15 à 40 % en poids, sur la base des dispersions de cire.

**4.** Dispersions aqueuses selon la revendication 1, **caractérisées en ce qu'**elles contiennent des émulsifiants non ioniques (b) sélectionnés dans le groupe constitué de monoesters de glycérol d'acides gras linéaires saturés et/ou insaturés, en particulier le monostéarate de glycérol et/ou le mono-oléate de glycérol,
et/ou **caractérisées en ce qu'**elles contiennent des émulsifiants non ioniques (b) dans une plage quantitative de 0,5 à

5,0 % en poids, sur la base des dispersions de cire.

5. Dispersions aqueuses selon la revendication 1, **caractérisées en ce qu'**elles contiennent des tensioactifs amphotères et/ou zwitterioniques (c) sélectionnés dans le groupe constitué de bétaïnes et/ou d'amphoacétates, et/ou **caractérisées en ce qu'**elles contiennent des tensioactifs amphotères et/ou zwitterioniques (c) dans une plage quantitative de 10 à 25 % en poids, sur la base des dispersions de cire.

6. Dispersions aqueuses selon la revendication 1, **caractérisées en ce qu'**elles contiennent des tensioactifs non ioniques additionnels (d) différents de (b), sélectionnés dans le groupe constitué de polyglucosides alkyle et/ou alcényle et de glucamides alkyle et/ou alcényle,
et/ou **caractérisées en ce qu'**elles contiennent des tensioactifs non ioniques additionnels (d) différents de (b) dans une plage quantitative de 0 à 5 % en poids, sur la base des dispersions de cire.

7. Dispersions aqueuses selon la revendication 1, **caractérisées en ce qu'**elles contiennent d'autres auxiliaires ou additifs (e), y compris des sels, dans une plage quantitative de 0,1 à 15 % en poids, sur la base des dispersions de cire,
et/ou **caractérisées en ce qu'**elles contiennent de l'eau (f) en une quantité allant jusqu'à 100 % en poids, sur la base des dispersions de cire.

8. Dispersions aqueuses selon la revendication 1, **caractérisées en ce qu'**elles contiennent

a) une cire naturelle sélectionnée dans le groupe constitué d'huile de colza hydrogénée comprenant au moins 90 % en poids, sur la base de l'huile de colza hydrogénée, de triglycérides de mélanges d'acides gras comprenant 0 à 10 % en poids d'acide gras C16, 35 à 45 % en poids d'acide gras C18, 5 à 15 % en poids d'acide gras C20 et 40 à 50 % en poids d'acide gras C22,
b) des émulsifiants non ioniques sélectionnés dans le groupe constitué de monoesters de glycérol d'acides gras, en particulier le monostéarate de glycérol et/ou le mono-oléate de glycérol,
c) des tensioactifs amphotères et/ou zwitterioniques sélectionnés dans le groupe constitué de bétaïnes et/ou d'amphoacétates, et
d) des tensioactifs non ioniques optionnels différents de (b), sélectionnés dans le groupe constitué de polyglucosides alkyle/alcényle et de glucamides alkyle/alcényle,
(e) d'autres auxiliaires ou additifs, y compris des sels, et
(f) de l'eau,

ou **caractérisées en ce qu'**elles contiennent

a) 15 à 40 % en poids d'une cire naturelle sélectionnée dans le groupe constitué d'huile de colza hydrogénée comprenant au moins 90 % en poids, sur la base de l'huile de colza hydrogénée, de triglycérides de mélanges d'acides gras comprenant 0 à 10 % en poids d'acide gras C16, 35 à 45 % en poids d'acide gras C18, 5 à 15 % en poids d'acide gras C20 et 40 à 50 % en poids d'acide gras C22,
b) 0,5 à 5,0 % en poids d'émulsifiants non ioniques sélectionnés dans le groupe constitué de monoesters de glycérol d'acides gras, en particulier le monostéarate de glycérol et/ou le mono-oléate de glycérol,
c) 10,0 à 25,0 % en poids de tensioactifs amphotères et/ou zwitterioniques sélectionnés dans le groupe constitué de bétaïnes et/ou d'amphoacétates, en particulier la cocoamidopropyl bétaïne,
d) 0,0 à 5,0 % en poids de tensioactifs non ioniques différents de (b), sélectionnés dans le groupe constitué de polyglucosides alkyle/alcényle et de glucamides alkyle/alcényle,
e) 0,1 à 15 % en poids d'autres auxiliaires ou additifs, y compris des sels, en particulier des conservateurs et/ou des ajusteurs de pH et/ou des sels, et
(f) jusqu'à 100 % en poids d'eau, sur la base des dispersions de cire.

9. Dispersions aqueuses de cire selon la revendication 1, **caractérisées en ce qu'**elles sont essentiellement exemptes de composants contenant une ou plusieurs unités d'oxyde d'éthylène et/ou d'oxyde de propylène.

10. Dispersions aqueuses selon la revendication 1, **caractérisées en ce qu'**elles consistent en :

a) 15 à 40 % en poids d'une cire naturelle sélectionnée dans le groupe constitué d'huile de colza hydrogénée comprenant au moins 90 % en poids, sur la base de l'huile de colza hydrogénée, de triglycérides de mélanges d'acides gras comprenant 0 à 10 % en poids d'acide gras C16, 35 à 45 % en poids d'acide gras C18, 5 à 15 % en poids d'acide gras C20 et 40 à 50 % en poids d'acide gras C22,

b) 0,5 à 5,0 % en poids d'émulsifiants non ioniques sélectionnés dans le groupe constitué de monoesters de glycérol d'acides gras, en particulier le monostéarate de glycérol et/ou le mono-oléate de glycérol,
c) 10,0 à 25,0 % en poids de tensioactifs amphotères et/ou zwitterioniques sélectionnés dans le groupe constitué de bétaïnes et/ou d'amphoacétates, en particulier la cocoamidopropyl bétaïne,
(e) 0,1 à 15 % en poids d'autres auxiliaires ou additifs, y compris des sels, en particulier des conservateurs et/ou des ajusteurs de pH et/ou des sels, et
(f) jusqu'à 100 % en poids d'eau, sur la base des dispersions de cire.

**11.** Dispersions aqueuses de cire selon la revendication 1, **caractérisées en ce que** la cire naturelle, à savoir l'huile de colza hydrogénée, présente une taille moyenne de particules d50 - mesurée par diffraction laser au moyen d'un Mastersizer 2000® - comprise entre 0,1 et 1,0 μm, en particulier entre 0,1 et 0,2 μm.

**12.** Procédé de préparation de dispersions aqueuses de cire selon la revendication 1, **caractérisé en ce que**, après addition des composants (a), (b) et (c) et/ou optionnellement (d) à une partie de la quantité totale d'eau (f), le mélange est chauffé à environ 85 à 95 °C sous agitation, refroidi à environ 40 à 60 °C avant l'addition du composant (e) et de la partie restante d'eau (f).

**13.** Utilisation de dispersions aqueuses de cire selon la revendication 1 ou 12 en tant qu'agent perlant dans des compositions de soins personnels pour le traitement des cheveux et/ou du corps, notamment en des quantités de 0,1 à 5 % en poids, calculées en tant que dispersions aqueuses de cire et sur la base de la composition de soins personnels,

ou utilisation de dispersions aqueuses de cire selon la revendication 1 ou 12 en tant qu'agent conditionneur dans des compositions de soins personnels pour le traitement des cheveux, notamment pour l'amélioration du démêlage à sec et/ou humide et/ou de la casse des cheveux, de préférence en des quantités de 0,1 à 5 % en poids, calculées en tant que dispersions aqueuses de cire et sur la base de la composition de soins personnels,
ou utilisation de dispersions aqueuses de cire selon la revendication 1 ou 12 en tant qu'agent conditionneur dans des compositions de soins personnels pour le traitement du corps,
notamment pour l'amélioration du ressenti sensoriel du corps, de préférence en des quantités de 0,1 à 5 % en poids, calculées en tant que dispersions aqueuses de cire et sur la base de la composition de soins personnels.

**14.** Utilisation de dispersions aqueuses de cire selon la revendication 13 dans des compositions de soins personnels pour le traitement des cheveux et/ou du corps, essentiellement exemptes de composants contenant une ou plusieurs unités d'oxyde d'éthylène et/ou d'oxyde de propylène,
ou utilisation de dispersions aqueuses de cire selon la revendication 13 dans des compositions de soins personnels pour le traitement des cheveux et/ou du corps, essentiellement exemptes de tensioactifs contenant des groupes sulfate.

**15.** Compositions de soins personnels pour le traitement des cheveux et/ou du corps, comprenant :

A) 0,1 à 5 % en poids, en particulier 0,25 à 3 % en poids, des dispersions aqueuses de cire selon la revendication 1 ou obtenues selon le procédé de la revendication 12, calculées en tant que dispersions aqueuses de cire et sur la base de la composition de soins personnels, et
B) un ou plusieurs tensioactifs détergents sélectionnés dans les groupes constitués de tensioactifs anioniques, tensioactifs non ioniques, tensioactifs amphotères ou zwitterioniques, et
C) des polymères cationiques optionnels, et
D) d'autres auxiliaires ou additifs optionnels comprenant des sels et de l'eau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03003991 A
- WO 2020229097 A
- US 20110247644 A
- US 4380646 A
- WO 9703043 A
- WO 02090369 A